(19) Europäisches Patentamt
European Patent Office
Office européen des brevets

(11) Veröffentlichungsnummer: **0 280 951**
**A1**

(12) # EUROPÄISCHE PATENTANMELDUNG

(21) Anmeldenummer: **88102169.5**

(22) Anmeldetag: **15.02.88**

(51) Int. Cl.[4]: **C07C 103/50**, C07D 209/08, C07D 209/34, C07D 211/46, A61K 31/16, A61K 31/40

(30) Priorität: **21.02.87 DE 3705622**

(43) Veröffentlichungstag der Anmeldung: **07.09.88 Patentblatt 88/36**

(84) Benannte Vertragsstaaten: **AT BE CH DE ES FR GB GR IT LI LU NL SE**

(71) Anmelder: **BOEHRINGER MANNHEIM GMBH**
**Sandhofer Strasse 116**
**D-6800 Mannheim 31(DE)**

(72) Erfinder: **Simon, Herbert, Dr. rer. nat.**
**Händelstrasse 5**
**D-6840 Lampertheim(DE)**
Erfinder: **Michel, Helmut**
**Ziegelgasse 2a**
**D-6800 Mannheim 31(DE)**
Erfinder: **Bartsch, Wolfgang, Dr. med. vet.**
**Franconviller-Strasse 5**
**D-6806 Viernheim(DE)**

(54) **Neue Amino-propanol-Derivate, Verfahren zu ihrer Herstellung und diese Verbindungen enthaltende Arzneimittel sowie Zwischenprodukte.**

(57) Die vorliegende Erfindung betrifft Amino-propanol-Derivate der allgemeinen Formel I

$$Ar-O-CH_2-\underset{\displaystyle |}{\overset{OH}{CH}}-CH_2-NH-A-X-\underset{R}{\underset{|}{N}}-B-ONO_2 \qquad (I)$$

worin

Ar eine substituierte oder unsubstituierte aromatische oder heteroaromatische Gruppe bedeutet,

A eine geradkettige oder verzweigte Alkylenkette von 1-8 C-Atomen, wobei eine $-CH_2$-Gruppe durch eine Cycloalkylengruppe von 3-7 C-Atomen ersetzt sein kann, bedeutet

X eine -C(=O)-, -S(=O)-oder $-S(=O)_2$-Gruppe bedeutet

B eine geradkettige, mono-oder bicyclische, gegebenenfalls verzweigte, gesaettigte oder ungesaettigte Alkylenkette von 1-12 C-Atomen, worin eine $-CH_2$-Gruppe durch eine Cycloalkylen-Gruppe von 3-7 C-Atomen ersetzt sein kann und/oder bis zu 2 $-CH_2$-Gruppen jeweils durch ein Sauerstoffatom oder Schwefelatom oder eine -S(=O)-oder $-S(=O)_2$-Gruppe ersetzt sein koennen, bedeutet

R Wasserstoff oder eine geradkettige oder verzweigte, gesaettigte oder ungesaettigte Alkyl-oder Nitroxyalkyl-Gruppe von 1-6 C-Atomen darstellt, oder R auch gemeinsam mit dem benachbarten Stickstoffatom und einer $-CH_2$-Gruppe der Kette B einen heteroaliphatischen Ring mit 4-6 C-Atomen aufspannen kann, bedeutet

sowie deren physiologisch vertraegliche Salze,

Verfahren zu deren Herstellung, sowie Arzneimittel, die diese Verbindungen enthalten, zur Behandlung und/oder Prophylaxe von Herz-und Kreislauferkrankungen

EP 0 280 951 A1

Gegenstand der vorliegenden Erfindung sind auch die neuen Zwischenprodukte der allgemeinen Formel II

$$Ar-O-CH_2-\overset{\displaystyle OH}{\overset{|}{C}}H-CH_2-NH-A-X-\underset{\displaystyle R}{\underset{|}{N}}-B-OH \qquad (II)$$

in der Ar, X, A, B und R die oben genannten Bedeutungen haben,
zur Herstellung von Verbindungen der allgemeinen Formel I, sowie Verfahren zur Herstellung dieser Zwischenprodukte.

# Neue Amino-propanol-Derivate, Verahren zu ihrer Herstellung und diese Verbindungen enthaltende Arzneimittel sowie Zwischenprodukte

Gegenstand der vorliegenden Erfindung sind neue Aminopropanol-Derivate der allgemeinen Formel I

$$Ar{-}O{-}CH_2{-}\overset{\displaystyle OH}{\overset{|}{C}}H{-}CH_2{-}NH{-}A{-}X{-}\underset{\displaystyle R}{\underset{|}{N}}{-}B{-}ONO_2 \qquad (I)$$

worin
Ar eine substituierte oder unsubstituierte aromatische oder heteroaromatische Gruppe bedeutet,
A eine geradkettige oder verzweigte Alkylenkette von 1-8 C-Atomen, wobei eine $-CH_2$-Gruppe durch eine Cycloalkylengruppe von 3-7 C-Atomen ersetzt sein kann, bedeutet
X eine $-C(=O)-$, $-S(=O)$-oder $-S(=O)_2$-Gruppe bedeutet
B eine geradkettige, mono-oder bicyclische, gegebenenfalls verzweigte, gesaettigte oder ungesaettigte Alkylenkette von 1-12 C-Atomen, worin eine $-CH_2$-Gruppe durch eine Cycloalkylen-Gruppe von 3-7 C-Atomen ersetzt sein kann und/oder bis zu 2 $-CH_2$-Gruppen jeweils durch ein Sauerstoffatom oder Schwefelatom oder eine $-S(=O)$-oder $-S(=O)_2$-Gruppe ersetzt sein koennen, bedeutet
R Wasserstoff oder eine geradkettige oder verzweigte, gesaettigte oder ungesaettigte Alkyl-oder Nitroxyalkyl-Gruppe von 1-6 C-Atomen darstellt, oder R auch gemeinsam mit dem benachbarten Stickstoffatom und einer $-CH_2$-Gruppe der Kette B einen heteroaliphatischen Ring mit 4-6 C-Atomen aufspannen kann, bedeutet
sowie deren physiologisch vertraegliche Salze.

Der Rest Ar kann eine Phenylgruppe darstellen, die unsubstituiert oder gegebenenfalls ein-oder mehrfach substituiert sein kann. Als Substituenten kommen in Frage: Halogeno-, Cyano-, Hydroxy-, Amino-, Formyl-, Nitro-, Carboxyl-, Carbamoyl-, Trifluormethyl-, Alkyl-, Alkenyl-, Alkinyl-, Cycloalkyl-, Bicycloalkyl-, Alkanoyl-, Alkoxy-, Hydroxyalkyl-, Alkoxyalkyl-, Alkoxyalkoxyalkyl-, Alkoxyalkoxy-, Alkenyloxy-, Alkenyloxyalkyl-, Alkinyloxy-, Alkinyloxyalkyl-, Cycloalkoxy-, Alkylthio-, Alkylthioalkyl-, Morpholino-, Acylamino-, Acylaminoalkyl-, Acyloxy-, Alkoxycarbonyl-, Cycloalkyloxycarbonyl-, Aminocarbonylamino-, Aminocarbonylaminoalkyl-, Alkylaminocarbonylamino-, Dialkylaminocarbonylamino-, Dialkylaminocarbonylaminoalkyl-, Dialkylaminocarbonylalkyl-, Dialkylaminocarbonylalkoxy-, wobei bei den letzteren 4 Gruppen die beiden endstaendigen Alkylgruppen zusammen mit dem Stickstoffatom auch eine cyclische Gruppe mit vier oder fuenf C-Atomen bilden koennen, Cycloalkylaminocarbonylamino-, Alkylaminocarbonylaminoalkyl-, Cycloalkylaminocarbonylaminoalkyl-, Alkoxycarbonylaminoalkyl-, Cycloalkoxycarbonylaminoalkyl-, Carbamoylalkyl-, Alkylaminocarbonylalkyl-, Cycloalkylaminocarbonylalkyl oder Alkylaminocarbonylalkoxy-Gruppen.

Der Rest Ar kann ferner eine Thiadiazolyl-, Naphthyl-, Indenyl-, Indolyl-, Indazolyl-, Imidazolyl-, Benzimidazolyl-, Benztriazolyl-, Benzofuranyl-, Benzodioxolyl-, Benzothiophenyl-, Benzthiazolyl-, Benzisothiazolyl-, Chinolyl-, Isochinolyl-, Benzodiazinyl-, Benzopyranyl-, Benzothiinyl-, Benzothiazinyl-, Benzothiadiazinyl-, Benzoxathiinyl-, Benzoxazolyl-, Benzisoxazolyl-, Benzoxazinyl-, Benzodioxinyl-oder Carbazolyl-Gruppe bedeuten, wobei eine oder mehrere Doppenbindungen hydriert sein koennen, und diese Gruppen unsubstituiert oder gegebenenfalls ein-oder mehrfach substituiert sein koennen. Als Substituenten kommen in Frage: Alkyl-, Cyano-, Hydroxyalkyl-, Hydroxy-, Oxo-, Formyl-, Alkanoyl-, Alkylcarbonylamino- und Alkoxycarbonyl-Gruppen sowie die Morpholino-Gruppe.

Die Phenylgruppe kann vorzugsweise einfach (insbesondere in o-Stellung und in p-Stellung, aber auch in m-Stellung) oder zweifach (insbesondere in 2,5-Stellung, aber auch z.B. in 2,3-, 2,4-, 3,4-oder 3,5-Stellung) substituiert sein; sie kann aber auch drei-(insbesondere in 3,4,5-Stellung, aber auch z.B. in 2,3,4-, 2,3,5-oder 2,4,5-Stellung), vier-(z.B. in 2,3,4,5-Stellung) oder fuenffach substituiert sein. Als Substituenten an der Phenylgruppe kommen insbesondere in Frage: F, Cl, Br, J; CN; OH; $NH_2$; $NH-CO-NH_2$; $NO_2$; COOH; $CONH_2$, $CF_3$; Alkyl mit 1 - 10, vorzugsweise 1 - 4 C-Atomen, vorzugsweise Methyl oder Ethyl, ferner z. B. n-Propyl, Isopropyl, n-Butyl, Isobutyl, sek-Butyl, tert.-Butyl, Pentyl wie n-Pentyl, Hexyl wie n-Hexyl, Heptyl wie n-Heptyl, Octyl wie n-Octyl, Nonyl wie n-Nonyl oder Decyl wie n-Decyl; Alkenyl mit bis zu 10, vorzugsweise 2-4 C-Atomen, z.B. Vinyl, Allyl, Propenyl, Isopropenyl, Butenyl wie 1-Buten-1-, -2-, -3-oder -4-yl, 2-Buten-1-yl, 2-Buten-2-yl, Pentenyl, Hexenyl oder Decenyl; Alkinyl mit bis zu 10, vorzugsweise 2-4 C-Atomen, z.B. Ethinyl, 1-Propin-1-yl, Propargyl, Butinyl wie 2-Butin-1-yl, Pentinyl, Decinyl; Cycloalkyl mit 3-8, vorzugsweise 5 oder 6 C-Atomen, vorzugsweise Cyclopentyl oder Cyclohexyl, ferner z.B. Cyclopropyl, Cyclobutyl, 1-, 2-oder 3-Methylcyclopentyl, 1-, 2-, 3-oder 4-Methylcyclohexyl, Cycloheptyl oder Cyclooctyl; Bicycloalkyl mit 4-11, vorzugsweise 7 C-Atomen, vorzugsweise exo-oder endo 2-Norbornyl, ferner z.B. 2-Isobornyl-oder 5-camphyl; Hydroxyalkyl mit 1-5, vorzugsweise 1-2 C-Atomen, vorzugsweise Hydroxymethyl und 1-oder 2-Hydroxyethyl, ferner z.B. 1-Hydroxy-prop-1-yl, 2-Hydroxy-prop-1-yl, 3-Hydroxy-prop-1-yl, 1-Hydroxy-prop-2-yl, 1-Hydroxy-but-1-yl, 1-Hydroxy-pent-1-yl, Alkanoyl mit 1-7, vorzugsweise 1-4 C-Atomen, vorzugsweise Formyl, Acetyl oder Propionyl, ferner z.B. Butyryl, Isobutyryl, Valeroyl, Caproyl, Heptanoyl; Alkoxy mit 1-10, vorzugsweise 1-4 C-Atomen, vorzugsweise Methoxy oder Ethoxy, ferner z.B. n-Propoxy, Isopropoxy, n-Butoxy, Isobutoxy, sek.-Butoxy, tert.-Butoxy, Pentyloxy, Hexyloxy, Heptyloxy, Octyloxy, Nonyloxy oder Decyloxy; Alkoxyalkyl mit bis zu 10, vorzugsweise 2-6 C-Atomen, z.B. Alkoxymethyl wie Methoxymethyl, Alkoxyethyl wie 1-oder 2-Methoxyethyl, 1-oder 2-n-Butoxyethyl, 1-oder 2-n-Octyloxyethyl; Alkoxyalkoxyalkyl mit bis zu 10, vorzugsweise 4-7 C-Atomen, z.B. Alkoxyalkoxymethyl wie 2-Methoxyethoxy-methyl, 2-Ethoxyethoxy-methyl oder 2-Iso-propoxyethoxymethyl, Alkoxyalkoxyethyl wie 2-(2-Methoxyethoxy)-ethyl oder 2-(2-Ethoxyethoxy)-ethyl; Alkoxyalkoxy mit bis zu 10, vorzugsweise 3-6 C-Atomen, z.B. 2-Methoxyethoxy, 2-Ethoxyethoxy oder 2-n-Butoxyethoxy; Alkenyloxy mit bix zu 10, vorzugsweise 2-4 C-Atomen, vorzugsweise Allyloxy, ferner z.B. Vinyloxy, Propenyloxy, Isopropenyloxy, Butenyloxy wie 1-Buten-1-, -2-, -3-oder 4-yloxy, 2-Buten-1-yloxy, 2-Buten-2-yloxy, Pentenyloxy, Hexenyloxy oder Decenyloxy; Alkenyloxylalkyl mit bis zu 10, vorzugsweise 3-6 C-Atomen, z.B. Allyloxymethyl; Alkinyloxy mit bis zu 10, vorzugsweise 2-4 C-Atomen, vorzugsweise Propargyloxy, ferner z. B. Ethinyloxy, 1-Propin-1-yloxy, Butinyloxy wie 2-Butin-1-yloxy, Pentinyloxy oder Decinyloxy; Alkinyloxyalkyl mit bis zu 10, vorzugsweise 3-6 C-Atomen, z.B. Ethinyloxymethyl, Propargyloxymethyl oder 2-(2-Butin-1-yloxy)-ethyl; Cycloalkoxy mit 3-8 vorzugsweise 5 oder 6 C-Atomen, vorzugsweise Cyclopentyloxy oder Cyclohexyloxy, ferner z.B. Cyclopropyloxy, Cyclobutyloxy, 1-, 2-oder 3-Methylcyclopentyloxy, 1-, 2-, 3-oder 4-Methylcyclohexyloxy, Cycloheptyloxy oder Cyclooctyloxy; Alkylthio mit 1-10, vorzugsweise 1-4 C-Atomen,vorzugsweise Methylthio oder Ethylthio, ferner z.B. n-Propylthio, Isopropylthio, n-Butylthio, Isobutylthio, sek.-Butylthio, tert.-Butylthio, Pentylthio, Hexylthio, Heptylthio, Octylthio, Nonylthio oder Decylthio; Alkylthioalkyl mit bis zu 10, vorzugsweise 2-6 C-Atomen, z.B. Methylthiomethyl, 2-Methyltioethyl oder 2-n-Butylthioethyl; Acylamino, vorzugsweise Alkanoylamino mit 1-7, vorzugsweise 1-4 C-Atomen wie Formylamino, Acetylamino, ferner Propionylamino, Butyrylamino, Isobutyrylamino, Valeroylamino, Caproylamino, Heptanoylamino, ferner auch Aroylamino wie Benzoylamino; Acylaminoalkyl, vorzugsweise Alkanoylaminoalkyl mit 1-8, vorzugsweise 3-6 C-Atomen wie Formylaminoethyl, Acetylaminoethyl, Propionylaminoethyl, n-Butyrylaminoethyl, Formylaminopropyl, Acetylaminopropyl, Propionylaminopropyl, Formylaminobutyl, Acetylaminobutyl, ferner Propionylaminobutyl, Butyrylaminobutyl; Acyloxy mit 1-6, vorzugsweise 2-4 C-Atomen, vorzugsweise Acetyloxy, Propionyloxy oder Butyryloxy, ferner z.B. Formyloxy, Valeroyloxy, Caproyloxy; Alkoxycarbonyl mit 1-5, vorzugsweise 2 und 3 C-Atomen, vorzugsweise Methoxycarbonyl oder Ethoxycarbonyl, ferner z.B. n-Propoxycarbonyl, Isopropoxycarbonyl, n-Butoxycarbonyl, Isobutoxycarbonyl, sek.-Butoxycarbonyl oder tert.-Butoxycarbonyl; Cycloalkoxycarbonyl mit 4-8, vorzugsweise 6 oder 7 C-Atomen, vorzugsweise Cyclopentyloxycarbonyl, Cyclohexyloxycarbonyl, ferner Cyclopropyloxycarbonyl, Cyclobutyloxycarbonyl oder Cycloheptyloxycarbonyl; Alkylaminocarbonylamino mit 2-4 C-Atomen wie Methylaminocarbonylamino, Ethylaminocarbonylamino, Propylaminocarbonylamino; Dialkylaminocarbonylamino mit 3-7, vorzugsweise 3-5 C-Atomen, vorzugsweise Dimethylaminocarbonylamino, Diethylaminocarbonylamino, ferner Di-n-propylaminocarbonylamino, Diisopropylaminocarbonylamino; (1-Pyrrolidino)-carbonylamino; (1-Piperidino) carbonylamino, Cycloalkylaminocarbonylamino mit 4-8, vorzugsweise 6 oder 7 C-Atomen, vorzugsweise Cyclopentylaminocarbonylamino, Cyclohexylaminocarbonylamino, ferner Cyclopropylaminocarbonylamino, Cyclobutylaminocarbonylamino, Cycloheptylaminocarbonylamino; Alkylaminocarbonylaminoalkyl mit 3-9, vorzugsweise 4-7 C-Atomen, vorzugsweise Methylaminocarbonylaminoethyl, Ethylaminocarbonylaminoethyl, Ethylaminocarbonylaminopropyl, Ethylaminocarbonylaminobutyl, ferner z.B. Methylaminocarbonylaminomethyl, n-Propylaminocarbonylaminobutyl, n-Butylaminocarbonylaminobutyl; Dialkylaminocarbonylaminoalkyl mit 4-11 C-Atomen, z.B. Dimethylaminocarbonylaminomethyl, Diethylaminocarbonylaminoethyl, Diethylaminocarbonylaminopropyl, Diethylaminocarbonylaminobutyl, (1-Pyrrolidino) carbonylaminoethyl, (1-Piperidino) carbonylaminoethyl; Cycloalkylaminocarbonylaminoalkyl mit 5-12, vorzugsweise 8-11 C-Atomen, vorzugsweise Cyclopentylaminocarbonylaminoethyl, Cyclopentylaminocarbonylaminopropyl, Cyclopentylaminocarbonylaminobutyl, Cyclohexylaminocarbonylaminoethyl, Cyclohexylaminocarbonylaminopropyl, Cyclohexylaminocarbonylaminobutyl, ferner z.B. Cyclopropylaminocarbonylaminomethyl, Cycloheptylaminocarbonylaminoethyl; Alkoxycarbonylaminoalkyl mit 3-12, vorzugsweise 4 - 9, C-Atomen vorzugsweise Methoxycarbonylaminoethyl, Ethoxycarbonylaminoethyl, n-Propoxycarbonylaminoethyl, Isopropoxycarbonylaminoethyl, n-Butoxycarbonylaminoethyl, Isobutoxycarbonylaminoethyl, sek.-Butylcarbonylaminoethyl, tert.-Butoxycarbonylaminoethyl, Ethoxycarbonylaminopropyl, n-Butoxycarbonylaminopropyl, Ethoxycarbonylaminobutyl, n-Butoxycarbonylaminobutyl, ferner z.B. n-Propoxycarbonylaminopropyl, n-Propoxycarbonylaminobutyl, Isopropoxycarbonylaminobutyl; Cycloalkoxycarbonylaminoalkyl mit 5-12, vorzugsweise 8-11 C-Atomen, vorzugsweise Cyclopentyloxycarbonylaminoethyl, Cyclopentyloxycarbonylaminopropyl, Cyclopentyloxycarbonylaminobutyl, Cyclohexyloxycarbonylaminoethyl, Cyclohexyloxycarbonylaminopropyl, Cyclohexyloxycarbonylaminobutyl, ferner z.B. Cyclopropyloxycarbonylaminomethyl, Cycloheptyloxycarbonylaminoethyl; Carbamoylalkyl mit 2-5, vorzugsweise 2 C-Atomen, vorzugsweise Carbamoylmethyl, ferner Carbamoylethyl, Carbamoylpropyl, Carbamoylbutyl; Alkylaminocarbonylalkyl mit 3-9, vorzugsweise 3-6 C-Atomen, vorzugsweise Methylaminocarbonylmethyl, Ethylaminocarbonylmethyl, n-Propylaminocarbonylmethyl, Isopropylaminocarbonylmethyl, n-Butylaminocarbonylmethyl, Isobutylaminocarbonylmethyl, sek.-Butylaminocarbonylmethyl, tert.-Butylaminocarbonylmethyl, ferner z.B. Ethylaminocarbonylethyl, Ethylaminocarbonylpropyl, Ethylaminocarbonylbutyl, n-Propylaminocarbonylbutyl, n-Butylaminocarbonylbutyl; Dialkylaminocarbonylalkyl mit 4-11, vorzugsweise 4-8 C-Atomen, vorzugsweise Dimethylaminocarbonylmethyl, Diethylaminocarbonylmethyl, Di-n-propylaminocarbonylmethyl, (1-Pyrrolidino)carbonylmethyl, (1-Piperidino)carbonylmethyl, ferner z.B. Diethylaminocarbonylethyl, (1-Piperidino)carbonylethyl, Diethylaminocarbonylpropyl, Diethylaminocarbonylbutyl; Cycloalkylaminocarbonylalkyl mit 5-12, vorzugsweise 7 oder 8 C-Atomen, vorzugsweise Cyclopentylaminocarbonylmethyl, Cyclohexylaminocarbonylmethyl, ferner z.B. Cyclopropylaminocarbonylmethyl, Cyclobutylaminocarbonylmethyl, Cycloheptylaminocarbonylmethyl, Cyclohexylaminocarbonylethyl, Cyclohexylaminocarbonylpropyl, Cyclohexylaminocarbonylbutyl; Alkylaminocarbonylalkoxy mit 3-10, vorzugsweise 3-5 C-Atomen, vorzugsweise Methylaminocarbonylmethoxy, ferner z.B. Methylaminocarbonylethoxy, Methylaminocarbonylpropoxy; Dialkylaminocarbonylalkoxy mit 4-10, vorzugsweise 4-7 C-Atomen, vorzugs weise Dimethylaminocarbonylmethoxy, Diethylaminocarbonylethoxy, (1-Piperidino)-carbonylmethoxy, Cycloalkylaminocarbonylalkoxy mit 5-11, vorzugsweise 7 und 8 C-Atomen, vorzugsweise Cyclopentylaminocarbonylmethoxy, Cyclohexylaminocarbonylmethoxy.

Der Rest Ar' kann ferner beispielsweise bedeuten: 1,2,5-Thiadiazol-3-yl, 4-(N-Morpholino)-1,2,5-thiadiazol-3-yl; 1-oder 2-Naphthyl; 1-, 2-, 3-, (bevorzugt) 4-, 5-, 6-oder 7-Indanyl; 1-Oxo-4-, -5-, -6-oder (bevorzugt) -7-Indanyl; Alkyl-1-oxo-indanyl, bevorzugt 1-Oxo-5-methyl-7-indanyl; 1-Hydroxy-4-, -5-, -6-oder (bevorzugt)-7-indanyl; 1-, 2-, 3-, (bevorzugt) 4-, 5-, 6-oder 7-Indenyl; 1-, 2-, 3-, 4-, (bevorzugt) 5-, 6-, 7-oder 8-Tetralyl; Oxo-tetralyl, bevorzugt 1-Oxo-5-tetralyl, ferner 2-, 3-oder 4-oxo-5-tetralyl oder 1-, 2-, 3-oder 4 -oxo-6-tetralyl; Hydroxy-tetralyl, (bevorzugt) 1-Hydroxy-5-tetralyl, ferner 2-, 3-oder 4-Hydroxy-5-tetralyl; (bevorzugt) 4-, 5-, 6-oder 7-Indolyl; Alkylindonyl, vorzugsweise Methylindolyl, z.B. 2-Methyl-4-indolyl, 3-Methyl-4-indolyl oder 6-Methyl-4-indolyl, ferner z.B. 2-Ethyl-4-indolyl oder 6-Ethyl-4-indolyl; Dialkylindolyl, vorzugsweise Dimethylindolyl, z.B. 2,3-Dimethyl-4-indolyl, 2,6-Dimethyl-4-indolyl, ferner z.B. 2-Methyl-3-ethyl-4-indolyl, 2-Ethyl-3-methyl-4-indolyl, 2,3-Diethyl-4-indolyl; Cyanoindolyl, z.B. 2-Cyano-4-indolyl, 3-Cyano-4-indolyl; Alkyl-cyano-indolyl, bevorzugt 2-Cyano-6-methyl-4-indolyl, ferner z.B. 3-Cyano-6-methyl-4-indolyl; Carbamoylindolyl, vorzugsweise 2-Carbamoyl-4-indolyl, 3-Carbamoyl-4-indolyl, ferner z.B. 6-Carbamoyl-4-indolyl; Alkyl-carbamoyl-indolyl, vorzugsweise Methyl-carbamoyl-indolyl, z.B. 2-Carbamoyl-6-methyl-4-indolyl; Hydroxyalkyl-indolyl, bevorzugt 2-Hydroxymethyl-4-indolyl, ferner z.B. 2-Hydroxymethyl-5-indolyl, 3-Hydroxymethyl-4-indolyl, 2-(2-Hydroxyethyl)-4-indolyl; 2-Oxo-indolinyl, bevorzugt 2-Oxo-indolin-4-yl, ferner 2-Oxo-indolin-5-yl; Alkyl-2-oxo-indolinyl, bevorzugt Methyl-2-oxo-indolin-4-yl, z.B. 3-Methyl-2-oxo-indolin-4-yl, ferner z.B. 3-Ethyl-2-oxo-indolin-4-yl, 3-Isopropyl-2-oxo-indolin-4-yl; Dialkyl-2-oxo-indolinyl, z.B. 3,3-Dimethyl-2-oxo-indolin-4-yl, 3,3-Diethyl-2-oxo-indolin-4-yl; Indazol-(bevorzugt)-4-, -5-, -6-oder -7-yl; Benzimidazol-4-yl; Alkyl-benzimidazol-4-yl, bevorzugt Methyl-benzimidazol-4-yl, z.B. 3-Methyl-benzimidazol-4-yl, 1-Methyl benzimidazol-4-yl, 2-Methyl-benzimidazol-4-yl, 6-Methyl-benzimidazol-4-yl, 7-Methyl-benzimidazol-4-yl; Benzimidazolin-2-on-4-yl (bevorzugt), Benzimidazolin-2-on-5-yl; Alkylbenzimidazolin-2-on-4-yl, bevorzugt Methyl-benzimidazolin-2-on-4-yl, z.B. 6-Methyl-benzimidazolin-2-on-4-yl, 7-Methyl-benzimidazolin-2-on-4-yl, Benztriazol (bevorzugt) 4-oder -5-yl; Benzofuran-(bevorzugt) 4-, -5-, -6-oder -7-yl; Alkyl-benzofuran-4-yl, z.B. 2-Methyl-benzofuran-4-yl, 3-Methyl-benzofuran-4-yl, 6-Methylbenzofuran-4-yl; Alkanoyl-benzofuran-4-yl; z.B. 2-Acetyl-benzofuran-4-yl, 6-Acetyl-benzofuran-4-yl; Bis-alkanoyl-benzofuranyl, z.B. 2,4-Diacetyl-benzofuran-5-yl, 2,6-Diacetyl-benzofuran-4-yl; 1,3-Benzodioxolyl, bevorzugt 1,3-Benzodioxol-4-yl; Alkyl-1,3-benzodioxolyl, bevorzugt 2-Methyl-1,3-benzodioxol-4-yl, ferner z.B. 6-Methyl-1,3-benzodioxol-4-yl; Dialkyl-1,3-benzodioxolyl, bevorzugt 2,2-Dimethyl-1,3-benzodioxol-4-yl, ferner z.B. 2,2-Diethyl-1,3-benzodioxol-4-yl, 2,6-Dimethyl-1,3-benzodioxol-4-yl; 1,2-Benzisoxazol-(bevorzugt) 4-, -5-, -6-oder -7-yl; Alkyl-1,2-benzisoxazolyl, bevorzugt 3-Methyl-1,2-benzisoxazol-4-yl, ferner z.B. 3-Ethyl-1,2-benzisoxazol-4-yl, 3-Propyl-1,2-benzisoxazol-4-yl, 3-Isopropyl-1,2-benzisoxazol-4-yl, 6-Methyl-1,2-benzisoxazol-4-yl; 1,3-Benzoxazol-(bevorzugt) 4-, -5-, -6-oder -7-yl; Alkyl-1,3-benzoxazolyl, bevorzugt 2-Methyl-1,3-benzoxazol-4-yl, ferner z.B. 2-Ethyl-1,3-benzoxazol-4-yl, 6-Methyl-1,3-benzoxazol-4-yl, 6-Methyl-1,3-benzoxazol-4-yl; Aryl-1,3-benzoxazolyl, bevorzugt 2-Phenyl-1,3-benzoxazol-4-yl, 2-(4-Pyridyl)-1,3-benzoxazol-4-yl; Benzthiphen-(bevorzugt) 4-, -5-, -6-oder -7-yl; 1,2-Benzisothiazol-(bevorzugt) 4-, -5-, -6-oder -7-yl; Alkyl-1,2-benzisothiazol-yl, z.B. 6-Methyl-1,2-benzisothiazol-4-yl; 1,3-Benzthiazol-4-, -5-, -6-oder -- (bevorzugt) 7-yl; Alkyl-1,3-benzthiazol-7-yl, z.B. 2-Methyl-1,3-benzthiazol-7-yl; 4-Methyl-1,3-benzthiazol-7-yl, 2-Ethyl-1,3-benzthiazol-7-yl; 2-Aryl-1,3-benzthiazol-7-yl, z.B. 2-Phenyl-1,3-benzthiazol-7-yl; 2-(4-Chlorphenyl)-1,3-benzthiazol-7-yl; 2-(4-Pyridyl)-1,3-benzthiazol-7-yl; 1,2 Dihydro-2-oxo-3-, -4-, (bevorzugt) -5-, -6-, -7-oder -8-chinolyl; 1,2,3,4-Tetrahydro-(bevorzugt) -5-, -6-, -7-oder -8-chinolyl; 1,2,3,4-Tetrahydro-2-oxo (bevorzugt) -5-, -6-, -7-oder -8-chinolyl; 1,2-Dihydro-8-hydroxy-2-oxo-(bevorzugt) 5-, -6-oder -7-chinolyl; 1,2-Dihydro-8-alkoxy-2-oxo-(bevorzugt) 5-, -6-oder -7-chinolyl, z.B. 1,2-Dihydro-8-methoxy-2-oxo-5-chinolyl; 1,2,3,4-Tetrahydro-8-hydroxy-2-oxo-(bevorzugt) 5-, -6-, oder -7-chinolyl; 1,2,3,4-Tetrahydro-8-alkoxy-2-oxo-(bevorzugt) 5-, -6-oder -7-chinolyl, z.B. 1,2,3-4-Tetrahydro-8-methoxy-2-oxo-5-chinolyl; 1,2,3,4-Tetrahydro-8-alkanoylamino-2-oxo-(bevorzugt) 5-, -6-oder -7-chinolyl, z.B. 1,2,3,4-Tetrahydro-8-acetylamino-2-oxo-5-chinolyl; 1,2-Dihydro-3-cyano-2-oxo-(bevorzugt) 5-, -6-, -7-oder -8-chinolyl; 1,2-Dihydro-3-cyano-2-oxo-7-methyl-5-chinolyl; 1,2-Dihydro-1-oxo-(bevorzugt) 4-, -5-, -6-, -7-oder -8-isochinolyl, 1,2-Dihydro-2-alkyl-1-oxo-(bevorzugt) 4-, -5-, -6-, -7-oder -8-isochinolyl, z.B. 1,2-Dihydro-2-methyl-1-oxo-4-isochinolyl; 1,2,3,4-Tetrahydro-2-alkanoyl-(bevorzugt)-5-, -6-, -7-oder -8-isochinolyl, bevorzugt 1,2,3,4-Tetrahydro-2-formyl-5-isochinolyl, ferner z.B. 1,2,3,4-Tetrahydro-2-acetyl-5-isochinolyl; 1,2-Dihydro-2-oxo-1,3-benzodiazin-(bevorzugt) 5-, -6-, -7-oder -8-yl; 2H-3,4-Dihydro-5-, -6-, -7-oder -(bevorzugt) -8-benzopyranyl; 2H-5-, 6-, 7-oder -(bevorzugt) -8-benzopyranyl; 2H-2-oxo-5-alkyl-7-oder-(bevorzugt) 8-benzopyranyl, z.B. 2H-2-oxo-5-methyl-8-benzopyranyl; 2H-3-Cyano-5-, -6-, -7-oder -(bevorzugt) 8-benzopyranyl; 2H-3,4-Dihydro-5-, -6-, -7-oder -(bevorzugt) -8-benzothiinyl, 3,4-Dihydro-1H-2,2-dioxo-2,1-benzothiazin-(bevorzugt) 5-, -6-, -7-oder -8-yl; 3,4-Dihydro-1H-1--alkyl-2,2-dioxo-2,1-benzothiazin-(bevorzugt) 5-, -6-, -7-oder -8-yl, z.B. 3,4-Dihydro-1H-1-methyl-2,2-dioxo-2,1-benzothiazin-5-yl; 3,4-Dihydro-2H-3-oxo-1,4-benzothiazin-5-, -6-, -7-oder -- (bevorzugt) 8-yl; 5-oder 6-Alkyl-3,4-dihydro-3-oxo-1,4-benzothiazin-8-yl, z.B. 6-Methyl-3,4-dihydro-3-oxo-1,4-benzothiazin-8-yl; 1,1-Dioxo-1,2,4-benzothiadiazin-5-, -6-, -7-oder -8-yl; 1,1-Dioxo-3-alkyl-1,2,4-benzothiadiazin-5-, -6-, -7-oder -8-yl, z.B. 1,1-Dioxo-3-methyl-1,2,4-benzothiadiazin-5-, -6-, -7-oder -8-yl; 1,1-Dioxo-3-alkanoyl-1,2,4-benzothiadiazin-5-, -6-, -7-oder -8-yl, z.B. 1,1-Dioxo-3-formyl-1,2,4-benzothiadiazin-5-, -6-, -7-oder -8-yl, 1,1-Dioxo-3-acetyl-1,2,4-benzothiadiazin-5-, -6-, -7-oder -8-yl; 1,1-Dioxo-3-aroyl-1,2,4-benzothiadiazin-5-, -6-, -7-oder -8-yl; z.B. 1,1-Dioxo-3-benzoyl-1,2,4-benzothiadiazin-5-, -6-, -7-oder -8-yl, 1,1-Dioxo-3-(4-pyridyl-carbonyl)-1,2,4-benzothiadiazin-5-, -6-, -7-oder -8-yl; 3,4-Dihydro-2,2-dioxo-1,2-benzoxathiin-(bevorzugt) 5-, -6-, -7-oder -(bevorzugt) 8-yl; 1-, 2-, 3-oder (bevorzugt) 4-Carbazolyl.

A kann eine geradkettige Alkylenkette von 1-8, bevorzugt 1-3 C-Atomen, bevorzugt Methylen, Ethylen, Trimethylen, ferner Tetramethylen, Pentamethylen, Hexamethylen, Heptamethylen, Octamethylen bedeuten. Die Alkylenkette A kann auch verzweigt sein und aus 2 - 8, bevorzugt 2 - 5 C-Atomen bestehen. Bevorzgut sind z.B. Methylmethylen, 1-Methylethylen, Dimethylmethylen, 1,1-Dimethylethylen, 1,1-Dimethyl-trimethylen, 1-Methyl-trimethylen; ferner kommen z.B. 1-Ethyl-methylen, 1,1-Dimethyl-tetramethylen, 1,2-Dimethyl-trimethylen, 2,2-Dimethyl-trimethylen, 3,3-Dimethyl-trimethylen, 1,1-Dimethylpentamethylen, 1,1-Dimethyl-hexamethylen, 1-Methyl-tetramethylen in Frage.

Eine $-CH_2$-Gruppe oder Alkylenkette kann durch eine Cycloalkylen-Gruppe mit 3-7, vorzugsweise 6 C-Atomen ersetzt sein. Vorzugsweise kommen der 1,2-1,3-und 1,4-Cyclohexylen-Rest in Frage, wobei die Stellung der Substituenten jeweils cis oder trans sein kann. Ferner kann z.B. die Methylen-1,2-, -1,3-oder -1,4-cyclohexylen-Gruppe, die Methylen-1,2-, -1,3-oder -1,4-cyclohexylen-methylen-Gruppe, die Ethylen-1,2-, -1,3-oder -1,4-cyclohexylen-Gruppe, die Trimethylen-1,2-, -1,3-oder -1,4-cyclohexylen-Gruppe, die Tetramethylen-1,2-, -1,3-oder -1,4-cyclohexylen-Gruppe oder auch die Methylen-1,2-, -1,3-oder -1,4-cyclohexylen-ethylen-Gruppe oder die Ethylen-1,2-, -1,3-oder -1,4-cyclohexylen-tetramethylen-Gruppe eingesetzt werden, die Konfiguration der Cycloalkylen-Gruppe kann jeweils cis oder trans sein. Weitere Alkylenketten A sind z.B. die 1,2-Cyclopropylen-Gruppe, die Methylen-1,2-cyclopropylen-Gruppe, die Tetramethylen-1,2-cyclopropylen-Gruppe, die Methylen-1,2-cyclopropylen-methylen-Gruppe, die Methylen-1,2-cyclopropylen-ethylen-Gruppe, die 1,2-oder 1,3-Cyclobutylen-Gruppe, die Methylen-1,2-oder -1,3-cyclobutylen-Gruppe, die Tetramethylen-1,2-oder -1,3-cyclobutylen-Gruppe, die Methylen-1,2-oder -1,3-cyclobutylen-methylen-Gruppe, die Methylen-1,2-oder -1,3-cyclobutylen-ethylen-Gruppe, die 1,2-oder 1,3-Cyclopentylen-Gruppe, die Methylen-1,2-oder -1,3-cyclopentylen-Gruppe, die Tetramethylen-1,2-oder -1,3-cyclopentylen-Gruppe, die Methylen-1,2-oder -1,3-cyclopentylen-methylen-Gruppe, die Methylen-1,2-oder -1,3-cyclopentylen-ethylen-Gruppe, die 1,2-1,3-oder 1,4-Cycloheptylen-Gruppe, die Methylen-1,2-, -1,3-oder -1,4-cycloheptylen-Gruppe, die Tetramethylen-1,2-, -1,3-oder -1,4-cycloheptylen-Gruppe, die Methylen-1,2-, -1,3-oder -1,4-cycloheptylen-methylen-Gruppe, die Methylen-1,2-, -1,3-oder -1,4-cycloheptylen-ethylen-Gruppe, die Konfiguration der Cycloalkylen-Gruppe kann jeweils cis oder trans sein.

Die Gruppe B kann eine geradkettige oder verzweigte Alkylenkette von 1-12, vorzugsweise 2-6 C-Atomen, bedeuten, vorzugsweise Ethylen, 1-Methyl-ethylen, 2-Methyl-ethylen, Trimethylen, 1-Methyl-trimethylen, 3-Methyl-trimethylen, Pentamethylen, 1-Methyl-tetramethylen-, 1-Ethyl-trimethylen, Hexamethylen, 1-Methyl-pentamethylen, 1-Ethyl-tetramethylen, 1-n-Propyl-trimethylen, 1,1-Dimethyl-ethylen, ferner z.B. 1,1-Dimethyl-trimethylen, 2,2-Dimethyl-trimethylen oder 1,1-Dimethyl-hexamethylen.

Als cyclische oder bicyclische Alkylenkette B kommen vorzugsweise die 1,2-Cyclopentylen-Gruppe, die 1,2-1,3-oder 1,-4 Cyclohexylen-Gruppe, die Methylen-1,2-cyclohexylen-Gruppe oder der 2,6-Dioxabicyclo-[3.3.0]octan-4,8-ylen-Rest in Frage, wobei die Konfiguration der Cycloalkylen-Gruppe jeweils cis oder trans sein kann, ferner kommen z.B. die 1,2-, 1,3-oder 1,4-Phenylen-methylen-Gruppe, die Methylen-1,2-, -1,3-oder -1,4-Phenylen-methylen-Gruppe, die Ethylen-1,4-phenylen-Gruppe, die Ethylen-1,4-phenylen-ethylen-Gruppe, die . Ethylen-1,4-phenylen-tetramethylen-Gruppe oder die Trimethylen-1,4-phenylen-trimethylen-Gruppe in Frage.

R kann Wasserstoff oder einen geradkettigen oder verzweigten, ungesaettigten oder ungesaettigten Alkylrest von 1-6, vorzugsweise 1-3 C-Atomen bedeuten, vorzugsweise die Methyl-Gruppe, die Ethyl-Gruppe, die n-Propyl-Gruppe, die Isopropyl-Gruppe oder die Allyl-Gruppe, ferner z.B. die n-Butyl-Gruppe, die n-Pentyl-Gruppe und die n-Hexylgruppe, wobei in allen Faellen noch 1 H-Atom durch die $O\text{-}NO_2$-Gruppe ersetzt sein kann. Im Fall, daß R gemeinsam mit einer $-CH_2$-Gruppe der Kette B und dem Stickstoffatom einen heteroaliphatischen Ring von 4-6 C-Atomen aufspannen kann, kommen vorzugsweise der Pyrrolidinylen-, der Piperidinylen-und der 1,2-, 1,3-oder 1,4-Piperidinylen-methylen-Rest in Frage, ferner z.B. der Perhydroazepinylen-Rest.

Verbindungen ähnlicher Art sind bereits z. B. beschrieben in den Patentanmeldungen EP 117 089, SE 385693 und DE 2418030.

Die dortigen Verbindungen unterscheiden sich jedoch dadurch von den erfindungsgemaeßen Verbindungen der allgemeinen Formel I, daß sie nicht die Gruppierung $-O\text{-}NO_2$ besitzen. Die erfindungsgemaeßen Verbindungen der allgemeinen Formel I besitzen wertvolle Eigenschaften. Sie besitzen nicht nur $\beta$-Rezeptoren blockierende Aktivitaet, sondern sie bewirken auch eine Verminderung des Sauerstoffbedarfs des Herzens, eine Erhoehung des Blutflusses und eine Erniedrigung des Blutdruckes. Sie eignen sich daher zur Prophylaxe und/oder Behandlung von Herz-und Kreislauferkrankungen, wie z.B. Hochdruck und Angina pectoris.

Die erfindungsgemaeßen Verbindungen werden ueblicherweise in Mengen von 20-500 mg pro Tag bezogen auf 75 kg Koerpergewicht appliziert. Bevorzugt ist es, 2-3 mal pro Tag 1-2 Tabletten mit einem Wirkstoffgehalt von 10-200 mg zu verabreichen. Die Tabletten koennen auch retardiert sein, wodurch nur noch 1 mal pro Tag 1-2 Tabletten mit 20-500 mg Wirkstoff gegeben werden muß. Der Wirkstoff kann auch durch Injektion 1-8 mal pro Tag bzw. durch Dauerinfusion gegeben werden, wobei Mengen von 5-200 mg/Tag normalerweise ausreichen.

Die erfindungsgemaeßen Verbindungen der allgemeinen Formel I koennen in an sich bekannter Weise dadurch hergestellt werden, daß man

a) eine Verbindung der allgemeinen Formel II

$$Ar\text{-}O\text{-}CH_2\text{-}\overset{OH}{\overset{|}{C}}H\text{-}CH_2\text{-}NH\text{-}A\text{-}X\text{-}\underset{R}{\underset{|}{N}}\text{-}B\text{-}OH \qquad (II)$$

in der A, Ar, B, X und R die oben angegebenen Bedeutungen besitzen, einer Nitratester-Bildungsreaktion unterwirft, oder

b) eine Verbindung der allgemeinen Formel III

Ar'-O-Z (III)

in der Ar' die gleiche Bedeutung wie Ar hat oder gegebenenfalle eine entsprechende Synthesevorstufe sein kann und Z eine der Gruppierungen

$$-CH_2\text{-}\overset{O}{\widehat{CH\text{-}CH_2}} \qquad \text{oder} \qquad -CH_2\text{-}\overset{OH}{\overset{|}{C}}H\text{-}CH_2\text{-}Y$$

darstellt, worin Y eine reaktive Gruppe bedeutet,

b1) mit einem Amin der allgemeinen Formel IV

$$H_2N\text{-}A\text{-}X\text{-}\underset{R}{N}\text{-}B\text{-}ONO_2 \qquad (IV)$$

in der A, B, X und R die oben angegebenen Bedeutungen haben, oder

b2) mit einem Amin der allgemeinen Formel V

$H_2N$-A-X-U (V)

worin A und X die oben angegebenen Bedeutungen haben und U eine nucleofuge Gruppe ist zu einer Verbindung der allgemeinen Formel VI

OH

Ar'-O-$CH_2$- CH -$CH_2$-NH-A-X-U (VI)

umsetzt und diese, gegebenenfalls nach Ueberfuehrung der Gruppe Ar' in die Gruppe Ar mit einer Verbindung der allgemeinen Formel VII

H N -B-$ONO_2$ (VIII)

R

in der B und R die oben angegebenen Bedeutungen besitzen, umsetzt, oder

c) eine Verbindung der allgemeinen Formel VIII

Ar'-OH (VIII)

in der Ar' die oben angegebenen Bedeutungen hat

c1) mit einer Verbindung der allgemeinen Formel IX

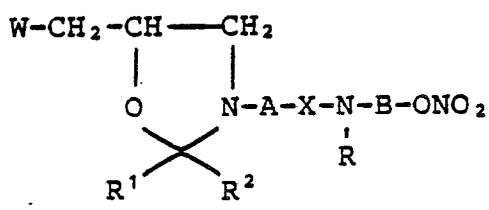

(IX)

worin A, B, X und R die oben angegebenen Bedeutungen besitzen, W Mesyloxy, Tosyloxy oder Halogen, $R_1$ Wasserstoff oder Alkyl und $R^2$ unabhaengig Wasserstoff, Alkyl oder Phenyl bedeuten oder $R^1$ und $R^2$ zusammen mit dem benachbarten Kohlenstoffatom ein Carbonyl-Radikal bilden, umsetzt, und gegebenenfalls die Gruppe Ar' in die Gruppe Ar ueberfuehrt und den Oxazolidin-Ring spaltet, oder

c2) mit einer Verbindung der allgemeinen Formel X

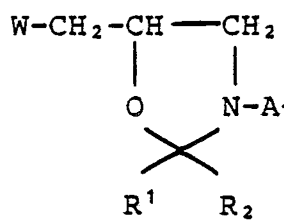

(X)

worin A, $R^1$, $R^2$, X, U und W die oben genannten Bedeutungen haben, umsetzt, den Oxazolidin-Ring spaltet und die erhaltene Verbindung der allgemeinen Formel VI, gegebenenfalls nach Uberfuehrung der Gruppe Ar' in die Gruppe Ar, mit einer Verbindung der allgemeinen Formel VII umsetzt, oder

c3) mit einer Verbindung der allgemeinen Formel XI

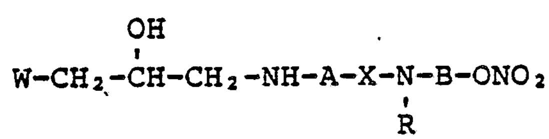

(XI)

in der A, B, W, X und R die oben angegebenen Bedeutungen besitzen, umsetzt und gegebenenfalls die Gruppe Ar' in die Gruppe Ar ueberfuehrt, oder

C4) mit einer Verbindung der allgemeinen Formel XII

OH

W-$CH_2$- CH -$CH_2$-NH-A-X-U (XII)

in der A, W, X und U die oben angegebenen Bedeutungen besitzen, zu einer Verbindung der allgemeinen

Formel VI umsetzt und diese, gegebenenfalls nach Ueberfuehrung der Gruppe Ar′ in die Gruppe Ar, mit einer Verbindung der allgemeinen Formel VII umsetzt.

Die Verbindungen der allgemeinen Formel II (Zwischenprodukte fuer die Herstellung von Verbindungen der allgemeinen Formel I) sind ebenfalls neu und Gegenstand dieser Anmeldung. Sie koennen hergestellt werden, indem man in an sich bekannter Weise

a) eine Verbindung der allgemeinen Formel III

a1) mit einem Amin der allgemeinen Formel XIII

$H_2N$-A-X- N -B-OH (XIII)
R

in der A, B, X und R die oben angegebenen Bedeutungen haben, umsetzt, oder

a2) mit einem Amin der allgemeinen Formel V zu einer Verbindung der allgemeinen Formel VI umsetzt und diese, gegebenenfalls nach Ueberfuehrung der Gruppe Ar′ in die Gruppe Ar, mit einer Verbindung der allgemeinen Formel XIV

H N -B-OH (XIV)
R

in der B und R die oben angegebenen Bedeutungen besitzen, umsetzt, oder

b) eine Verbindung der allgemeinen Formel VIII

b1) mit einer Verbindung der allgemeinen Formel XV

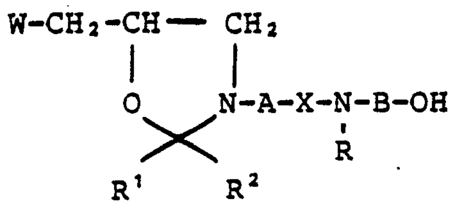

(XV)

in der A, B, $R^1$, $R^2$, W, X und R die oben angegebenen Bedeutungen haben, umsetzt und den Oxazolidin-Ring spaltet, oder

b2) mit einer Verbindung der allgemeinen Formel X zu einer Verbindung der allgemeinen Formel XVI

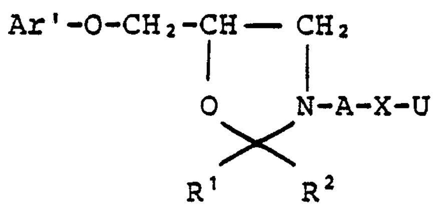

(XVI)

in der Ar′, $R^1$, $R^2$, A, X und U die oben angegebenen Bedeutungen haben, umsetzt und diese, gegebenenfalls vor oder nach Ueberfuehrung der Gruppe Ar′ in die Gruppe Ar, mit einer Verbindung der allgemeinen Formel XIV zu einer Verbindung der allgemeinen Formel XVII

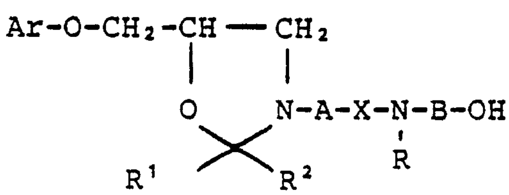

(XVII)

in der Ar, $R^1$, $R^2$, A, X, R und B die oben angegebenen Bedeutungen haben, umsetzt und dann den Oxazolidin-Ring spaltet, oder

b3) mit einer Verbindung der allgemeinen Formel X umsetzt, den Oxazolidin-Ring spaltet und die so erhaltene Verbindung der allgemeinen Formel VI, gegebenenfalls nach Ueberfuehrung der Gruppe Ar′ in die Gruppe Ar, mit einer Verbindung der allgemeinen Formel XIV umsetzt, oder

b4) mit einer Verbindung der allgemeinen Formel XVIII

$$W-CH_2-\overset{OH}{CH}-CH_2-NH-A-X-\underset{R}{N}-B-OH \quad (XVIII)$$

in der A, B, W, X und R die oben angegebenen Bedeutungen besitzen, umsetzt und gegebenenfalls die Gruppe Ar′ in die Gruppe Ar ueberfuehrt, oder

b5) mit einer Verbindung der allgemeinen Formel XII zu einer Verbindung der allgemeinen Formel VI umsetzt, und diese, gegebenenfalls nach Ueberfuehrung der Gruppe Ar′ in die Gruppe Ar, mit einer Verbindung der allgemeinen Formel XIV umsetzt,

c) eine Verbindung der allgemeinen Formel III mit Ammoniak oder einem geschuetzten Amin umsetzt, gegebenenfalls die Schutzgruppen abspaltet und die Gruppe Ar′ in die Gruppe Ar ueberfuehrt und die entstandene Verbindung XIX

$$Ar-O-CH_2-\overset{OH}{C}H-CH_2-NH_2 \quad (XIX)$$

in der Ar die angegebenen Bedeutung hat,

c1) mit einer Verbindung der allgemeinen Formel XX

$$H-A'-X-\underset{R}{N}-B-OH \quad (XX)$$

in der X, R und B die oben angegebenen Bedeutungen haben und A′ die gleiche Bedeutung wie A hat, wobei aber eine $-CH_2$-Gruppe in A′ durch eine C=O-Gruppe ersetzt ist, umsetzt und das entstandene Imin in situ oder anschließend reduziert, oder

c2) mit einer Verbindung der allgemeinen Formel XXI

H-A′-X-U (XXI)

in der A′, X und U die oben angegebenen Bedeutungen haben, umsetzt, das entstandene Imin in situ oder anschließend weiter zur Verbindung der allgemeinen Formel VI reduziert und diese mit einer Verbindung der allgemeinen Formel XIV umsetzt.

Die Amine IV koennen in an sich bekannter Weise dadurch hergestellt werden, daß man

a) Verbindungen der allgemeinen Formel V mit Verbindungen der allgemeinen Formel VII umsetzt, oder

b) Verbindungen der allgemeinen Formel V mit Verbindungen der allgemeinen Formel XIV zu Verbindungen der allgemeinen Formel XIII umsetzt und diese einer Nitratester-Bildungs reaktion unterwirft, oder

c) Verbindungen der allgemeinen Formel XXII

M-A-X-U (XXII)

in der A, X und U die oben angegebenen Bedeutungen haben und M eine Gruppe bedeutet, die in eine $-NH_2$-Gruppe ueberfuehrt werden kann, mit Verbindungen der allgemeinen Formel VII umsetzt und die Gruppe M in die $H_2N$-Gruppe ueberfuehrt, oder

d) Verbindungen der allgemeinen Formel XXII mit Verbindungen der allgemeinen Formel XIV zu Verbindungen der allgemeinen Formel XXIII

$$M-A-X-\underset{R}{N}-B-OH \quad (XXIII)$$

umsetzt und diese, nach Ueberfuehrung der Gruppe M in eine $-NH_2$-Gruppe, einer Nitratester-Bildungsreaktion unterwirft. Gegebenenfalls kann die Nitratester-Bildungsreaktion auch vor der Ueberfuehrung der Gruppe M in eine $-NH_2$-Gruppe durchgefuehrt werden.

e) Verbindungen der allgemeinen Formel XIV mit Verbindungen der allgemeinen Formel XXIV

Y′-A″-X-U (XXIV)

in der X und U die oben genannten Bedeutungen besitzen, Y′ die Bedeutung von Y hat oder Wasserstoff bedeuten kann und A″ die Bedeutung von A hat, jedoch eine geringere Anzahl an C-Atomen beinhaltet, umsetzt und die entstehenden Verbindungen der allgemeinen Formel XXV

$$Y'-A''-X-\underset{R}{N}-B-OH \quad (XXV)$$

in der Y′, A″, X, R und B die oben genannten Bedeutungen besitzen, mit Verbindungen der allgemeinen Formel XXVI

M′-A‴-Y″ (XXVI)

in der M′ die Bedeutung von M hat oder die $-NH_2$-Gruppe bedeuten kann, A‴ die Bedeutung von A″, und Y″ die Bedeutung von Y′ haben kann, zu Verbindungen der allgemeinen Formel XXIII

M-A‴-A″-X- N -B-OH (XXIII)
R

in der A‴ und A″ gemeinsam die Bedeutung von A haben umsetzt und diese wie unter d) weiter behandelt.

Die Nitratester-Bildungsreaktion der Verbindungen der allgemeinen Formeln II, XIII und XIV kann durchgeführt werden, indem die Verbindungen der allgemeinen Formel II, XIII und XIV mit einem nitratesterbildenden Reagenz, wie rauchender Salpetersäure, einer Mischung aus rauchender Salpetersäure und konz. Schwefelsäure bei niedrigen Temperaturen in Gegenwart oder Abwesenheit eines inerten Lösungsmittels umsetzt. Die Reaktionstemperaturen liegen zwischen Raumtemperatur und -60°C, bevorzugt zwischen -10°C und -30°C. Das Molverhältnis der Reaktionspartner liegt zwischen 1 und 10.

Alternativ kann die Nitratester-Bildungsreaktion durchgeführt werden, indem man in einer Verbindung der allgemeinen Formel II, XIII oder XIV selektiv eine aliphatische Hydroxylgruppe durch eine Halogengruppe ersetzt und anschließend das Reaktionsprodukt mit Silbernitrat in Gegenwart oder Abwesenheit eines Lösungsmittels bei Temperaturen zwischen Raumtemperatur und 100°C umsetzt. Das Molverhältnis der Umsetzungsreaktion zwischen der Halogenverbindung und Silbernitrat kann zwischen 1 und 10 liegen.

Die Umsetzungen der Verbindungen der allgemeinen Formel VIII mit Epihalogenhydrinen oder Verbindungen der allgemeinen Formeln IX, X, XI, XII, XV und XVIII werden in an sich bekannter Weise durchgeführt, indem man die Reaktionspartner in einem organischen Lösungsmittel und/oder Wasser in Gegenwart eines säurebindenden Mittels wie Alkalihydroxiden oder -hydriden oder organischen Stickstoffbasen bei Temperaturen zwischen Raumtemperatur und 100°C umsetzt. Die Molverhältnisse der Verbindungen der allgemeinen Formel VIII zu den Epihalogenhydrinen oder den Verbindungen der allgemeinen Formel IX, X, XI, XII, XV und XVIII können zwischen 1 und 100 liegen. Als organische Lösungsmittel kommen z. B. Methanol, Ethanol, Propanol, Benzol, Toluol, Tetrahydrofuran, Dimethylformamid oder Dimethylsulfoxid in Frage. Die Spaltung der Oxazolidin-Ringe in den Formeln IX, X, XV, XVI und XVII erfolgt unter sauren Bedingungen im Fall $R^2$, $R^3$ = H, Alkyl oder Aryl oder unter basischen Bedingungen (z. B. 4n NaOH/Ethanol) im Fall $R^2$, $R^3$ = $>$ CO.

Die Umsetzungen der Verbindungen der allgemeinen Formel III mit einem Amin der allgemeinen Formel IV, V und XIII oder einem geschützten Amin oder Ammoniak erfolgen in Gegenwart oder Abwesenheit eines Lösungsmittels zwischen 0° und 150°C, bevorzugt zwischen 20°C und 50°C. Als Lösungsmittel können z.B. Methanol, Ethanol, Propanol, iso-Propanol, Benzol, Toluol oder Dimethylformamid verwendet werden. Das molare Verhältnis der Reaktionspartner ist nicht kritisch. Es können Verhältnisse zwischen 1 und 100 gewählt werden. Gegebenenfalls können die Reaktionen unter erhöhtem Druck durchgeführt werden.

Die Gruppen M und M′ in Verbindungen der allgemeinen Formeln XXII, XXIII und XXVI können beispielsweise eine acylierte, vorzugsweise acetylierte, Gruppe -NH-Acyl, eine durch 1-oder 2 Benzylgruppen geschützte Gruppe $-NH-C_7H_7$ oder $-N(C_7H_7)_2$, eine Nitrogruppe $-NO_2$ oder Nitroso-Gruppe -NO sein oder auch mit dem benachbarten C-Atom zusammen eine -C≡N-Gruppe bilden. Die Demaskierung der -NH-Acyl-Gruppe erfolgt vorzugsweise mit anorganischen Säuren oder Basen, z. B. Salzsäure oder Natronlauge, in Gegenwart oder Abwesenheit eines organischen Lösungsmittels, z. B. Methanol oder Ethanol, bei Temperaturen zwischen Raumtemperatur und 150°C, die Molverhältnisse können zwischen 1 und 200 liegen. Die Abspaltung von Benzyl-Schutzgruppen erfolgt hydrogenolytisch in Gegenwart eines organischen Lösungsmittels und/oder Wasser sowie Palladium auf Kohle als Katalysator. Die Temperaturen können zwischen Raumtemperatur und 250°C, vorzugsweise Raumtemperatur und 60°C, liegen, der Wasserstoffdruck kann zwischen 1 und 300 bar vorzugsweise 1 bis 5 bar betragen.

Zur Reduktion der $-NO_2$-Gruppe kommen zahlreiche Verfahren, z.B. mit Zink in Salzsäure, mit Eisen in Salzsäure, mit Lithiumaluminiumhydrid in Ethern, mit anorganischen Sulfiden wie NaHS, $(NH_4)_2S$ oder $Na_2S_2O_4$ in wässriger und/oder alkoholischer Lösung, oder hydrogenolytisch mit Katalysatoren wie $PtO_2$, Pd, Raney-Nickel, vorzugsweise in einem alkoholischen Lösungsmittel wie Methanol und Ethanol, bei Drucken zwischen 1 und 200 bar, in Frage. Die Temperaturen können zwischen -20°C und +200°C, vorzugsweise zwischen Raumtemperatur und 100°C, liegen. Die Reduktion der C≡N-Gruppe zur $-CH_2-NH_2$-Gruppe kann z.B. mit Lithiumaluminiumhydrid in Ethern, Diboran in Ethern oder hydrogenolytisch mit Katalysatoren wie Raney-Nickel oder $PtO_2$ in organischen Lösungsmitteln und/oder Wasser bei Drucken zwischen 1 und 300 bar erfolgen. Die Temperaturen können zwischen -30°C und +200°C liegen.

Die Umsetzungen der Amine VII und XIV mit aktivierten Verbindungen der allgemeinen Formeln V, VI, XVI, XXII, XXIV und XXV werden in an sich bekannter Weise bevorzugt in einem Lösungsmittel wie Hexan, Diethylether, Tetrahydrofuran, Methylenchlorid, Benzol, Toluol oder Dimethylformamid oder in wässriger Lösung bei Temperaturen zwischen -50°C und +100°C, bevorzugt zwischen -30°C und Raumtemperatur, durchgeführt.

Im Fall

a) X = -C(=0)-

können die entsprechenden aktivierten Carbonsäuren z.B. in Form von Estern, Lactonen, Carbonsäurehalogeniden oder -anhydriden vorliegen, oder die Aktivierung der Carbonsäuren erfolgt durch aktivierende Reagenzien wie N, N'-Carbonyldiimidazol, N,N'-Dicyclohexylcarbodiimid, 1-Ethyl-3-(3-dimethylaminopropyl)carbodiimid, 1-Alkyl-2-halogen-pyridiniumsalzen o.ä. Die Aktivierung und die Umsetzung mit den Aminen VII und XIV kann dabei in einem Syntheseschritt durchgeführt werden. Es koennen molare Verhaeltnisse zwischen 1 und 100 gewaehlt werden. Gegebenenfalls koennen die Umsetzungen in Gegenwart einer Hilfsbase, vorzugsweise einem organischen Amin, durchgefuehrt werden,

b) X = $-\overset{O}{S}-$ oder

$$-\overset{O}{\underset{O}{S}}-$$

koennen die entsprechenden aktivierten Sulfinsaeuren bzw. Sulfonsaeuren z.B. in Form von Estern und Halogeniden, wobei diese aus geeigneten Vorstufen hergestellt werden, eingesetzt werden. Die Molverhaeltnisse zwischen den aktivierten Sulfinsaeuren bzw. Sulfonsaeuren und den Aminen VII und XIV koennen zwischen 1 und 100 liegen, vorzugsweise bei 1.

Die Umsetzungen der Verbindungen der allgemeinen Formel XXV mit Verbindungen der allgemeinen Formeln XXVI werden in an sich bekannter Weise durchgefuehrt, in dem man ein acides Proton in Verbindungen der allgemeinen Formel XXVI, mittels einer Base (z.B. Natriumhydrid, Kaliumcarbonat, Natriumhydroxid, Lithiumdiisopropylamid) in einem Loesungsmittel (z.B. Dimethylformamid, Methanol, Ethanol, Diethylether, Tetrahydrofuran, Toluol oder Wasser) abstrahiert und die entstandene Verbindung mit einer Verbindung der allgemeinen Formel XXV alkyliert. Als nucleofuge Gruppe Y' kommt vorwiegend ein Halogenidion oder ein Mesylat, Triflat, Tosylat, aber auch z.B. ein Acetat in Frage.

Die erfindungsgemäßen Verbindungen der allgemeinen Formeln I, II, III, VI, IX, X, XI, XII, XV, XVI, XVII, XVIII und XIX besitzen asymmetrische Kohlenstoffatome.

Gegenstand der Erfindung sind daher auch sämtliche möglichen Diastereomerengemische, Racemate und sämtliche optisch aktiven Formen der erfindungsgemäßen Verbindungen der allgemeinen Formeln I, II, VI, IX, X, XI, XII, XV, XVI, XVII, XVIII und XIX.

Zur Überführung der Verbindungen der allgemeinen Formeln I und II in ihre pharmakologisch unbedenklichen Salze setzt man diese, vorzugsweise in einem organischen Lösungsmittel, mit der äquivalenten Menge einer anorganischen oder organischen Säure, z.B. Salzsäure, Bromwasserstoffsäure, Salpetersäure, Phosphorsäure, Schwefelsäure, Ameisensäure, Essigsäure, Propionsäure, Oxalsäure, Fumarsäure, Maleinsäure, Bernsteinsäure, Adipinsäure, Benzoesäure, Salicylsäure, o-Acetoxybenzoesäure, Zimtsäure, Naphthoesäure, Mandelsäure, Citronensäure, Äpfelsäure, Weinsäure, Asparaginsäure, Glutaminsäure, Methansulfonsäure oder p-Toluolsulfonsäure um.

Die erfindungsgemäßen neuen Substanzen I und II und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat-und Citratpuffer, Ethanol, Komplexbildner (wie Ethylendiamintetraessigsäure und deren nicht toxische Salze), hochmolekulare Polymere (wie flüssiges Polyethylenoxid) zur Viskositätsregulierung. Feste Trägerstoffe sind z.B. Stärke, Lactose, Mannit, Methylcellulose, Talkum hochdispers Kieselgelsäuren, höhermolekulare Fettsäuren (wie Stearinsäure), Gelatine, Agar-Agar, Calciumphosphat, Magnesiumstearat, tierische und pflanzliche Fette und feste hochmolekulare Polymere (wie z.B. Polyethylenglykole), für orale Applikation geeignete Zubereitungen können gewünschtenfalls Geschmacks-und Süßstoffe enthalten.

Neben den nachfolgend aufgefuehrten Beispielen sind insbesondere die folgenden Verbindungen im Sinne der Anmeldung bevorzugt.

2-[2-Hydroxy-3-[4-(4-morpholino)-1,2,5-thiadiazol-3-yloxy]-propylamino]-N-(2-methyl-1-nitroxy-prop-2-yl)-essigsaeureamid

4-[2-Hydroxy-3-[4-(4-morpholino)-1,2,5-thiadiazol-3-yloxy]-propylamino]-4-methyl-N-(3-nitroxy-propyl)-valeriansaeureamid

2-[3-(2-Fluorophenoxy)-2-hydroxy-propylamino]-N-(2-nitroxy-propyl)-propionsaeureamid

trans-3-[3-(2-Fluorphenoxy)-2-hydroxy-propylamino]-3-methyl-N-(2-nitroxy-cyclohexyl)-buttersaeureamid
3-[2-Hydroxy-3-(2-methylphenoxy)-propylamino]-N-methyl-N-(3-nitroxy-propyl)-buttersaeureamid
trans-3-[2-Hydroxy-3-(2-methylphenoxy)-propylamino]-3-methyl-N-[(2-nitroxy-cyclohexyl)-methyl]-buttersaeureamid
4-[2-Hydroxy-3-(2-methylphenoxy)-propylamino]-4-methyl-N-(4-nitroxy-butyl)-valeriansaeureamid
2-[2-Hydroxy-3-(3-methylphenoxy)-propylamino]-2-methyl-N-(3-nitroxy-propyl)-propansulfonsaeureamid
2-[2-Hydroxy-3-[2-(2-norbornylexo)-phenoxy]-propylamino]-2-methyl-N-(3-nitroxy-propyl)-propionsaeureamid
4-[3-(4-Carbamoylmethyl-phenoxy)-2-hydroxy-propylamino]-4-methyl-N-(3-nitroxy-propyl)-valeriansaeureamid
N-(2,2-Dimethyl-3-nitroxy-propyl)-4-[2-hydroxy-3-[4-(2-methoxy-ethyl)-phenoxy]-propylamino]-4-methyl-valeriansaeureamid
4-[2-Hydroxy-3-[4-(2-methoxy-ethyl)-phenoxy]-propylamino]-3-methyl-buttersaeure-(4-nitroxy)-piperidid
2-[2-Hydroxy-3-[4-(2-methoxy-ethyl)-phenoxy]-propylamino]-2-methyl-N-(3-nitroxy-propyl)-propansulfonsaeureamid
cis-3-[2-Hydroxy-3-[(2-methylamino-carbonylmethoxy)-phenoxy]-propylamino]-3-methyl-N-(4-nitroxy-cyclohexyl)-buttersaeureamid
3-[3-(2-Allyloxy-phenoxy)-2-hydroxy-propylamino]-3-methyl-N-(1-nitroxy-but-3-yl)-buttersaeureamid
4-[3-(2-Allyloxy-phenoxy)-2-hydroxy-propylamino]-4-methyl-valeriansaeure-(4-nitroxymethyl)-piperidid
(2S)-2-[2-Hydroxy-3-[(2-propargyloxy)-phenoxy]-propylamino]-propionsaeure-(4-nitroxy)-piperidid
3-[2-Hydroxy-3-(2-methylmercapto-phenoxy)-propylamino]-3-methyl-N-(3-nitroxy-propyl)-buttersaeureamid
2-[2-Hydroxy-3-(2-methylmercapto-phenoxy)-propylamino]-2-methyl-propionsaeure-[4-(1-nitroxy-ethyl)]-piperidid
3-[3-(2,5-Dichloro-phenoxy)-2-hydroxy-propylamino]-3-methyl-N-(3-nitroxy-propyl)-buttersaeureamid
4-[3-(2,5-Dichloro-phenoxy)-2-hydroxy-propylamino]-4,N-dimethyl-N-(2-nitroxy-propyl)-valeriansaeureamid
2-[3-(2,5-Dichloro-phenoxy)-2-hydroxy-propylamino]-2-methyl-N-(3-methyl-1-nitroxy-but-3-yl)-propionsaeureamid
3-[3-(2-Chloro-5-methyl-phenoxy)-2-hydroxy-propylamino]-3-methyl-N-(3-nitroxy-propyl)-buttersaeureamid
4-[3-(2-Chloro-5-methyl-phenoxy)-2-hydroxy-propylamino]-4-methyl-valeriansaeure-(4-nitroxy)-piperidid
trans-2-[3-(2-Chloro-5-methyl-phenoxy)-2-hydroxy-propylamino]-2-methyl-N-[(2-nitroxy-cyclohexyl)-methyl]-propansulfonsaeureamid
3-[3-(4-Butanoylamino-2-cyano-phenoxy)-2-hydroxy-propylamino]-3,N-dimethyl-N-(3-nitroxy-propyl)-buttersaeureamid
3-[2-Hydroxy-3-(4-hydroxy-2,3,5-trimethyl-phenoxy)-propylamino]-3-methyl-N-(2,2-dimethyl-3-nitroxy-propyl)-buttersaeureamid
cis-2-[2-Hydroxy-3-(4-hydroxy-2,3,5-trimethyl-phenoxy)-propylamino]-N-(4-nitroxy-cyclohexyl)-propionsaeureamid
3-[2-Hydroxy-3-(4-methylcarbonyloxy-2,3,5-trimethyl-phenoxy)-propylamino]-3-methyl-N-(2,2-dimethyl-3-nitroxy-propyl)-buttersaeureamid
3-[2-Hydroxy-3-(4-methylcarbonyloxy-2,3,5-trimethyl-phenoxy)-propylamino]-3-methyl-N-(2-nitroxy-ethyl)-buttersaeureamid
3-[2-Hydroxy-3-(1-oxo-indan-7-yloxy)-propylamino]-3-methyl-N-(3-nitroxy-propyl)-buttersaeureamid
2-[2-Hydroxy-3-(1-oxo-indan-7-yloxy)-propylamino]-2,N-dimethyl-N-(2-nitroxy-ethyl)-propansulfonsaeureamid
4-[2-Hydroxy-3-(2-methyl-indol-4-yloxy)-propylamino]-N-methyl-N-(2-nitroxy-ethyl)-buttersaeureamid
trans-3-[2-Hydroxy-3-(2-methyl-indol-4-yloxy)-propylamino]-N-(2-nitroxy-cyclohexyl)-propionsaeureamid
3-[2-Hydroxy-3-(2-methyl-indol-4-yloxy)-propylamino]-3-methyl-buttersaeure-(4-nitroxy)-piperidid
2-[2-Hydroxy-3-(6-methyl-indol-4-yloxy)-propylamino]-essigsaeure-(4-nitroxy)-piperidid
3-[2-Hydroxy-3-(6-methyl-indol-4-yloxy)-propylamino]-N-(2,2-dimethyl-3-nitroxy-propyl)-propionsaeureamid
4-[3-(3-Cyano-indol-4-yloxy)-2-hydroxy-propylamino]-N-methyl-N-(2-nitroxy-ethyl)-buttersaeureamid
4-[2-Hydroxy-3-(3-methyl-1,2-benzisoxazol-4-yloxy)-propylamino]-4-methyl-valeriansaeure-(4-hydroxymethyl)-piperidid
4-[3-(1,2-Benzthiazol-4-yloxy)-2-hydroxy-propylamino]-4-methyl-N-(3-nitroxy-propyl)-valeriansaeureamid
3-[3-(1,3-Benzodioxol-4-yloxy)-2-hydroxy-propylamino]-3-methyl-N-(4-methyl-1-nitroxy-pent-4-yl)-buttersaeureamid
trans-3-[3-(2,2-Dimethyl-1,3-benzodioxol-4-yloxy)-2-hydroxy-propylamino]-3-methyl-N-(4-nitroxy-cyclohexyl)-buttersaeureamid
3,N-Dimethyl-N-(3-nitroxy-propyl)-3-[2-hydroxy-3-(1,2,3,4-tetrahydro-1-oxo-5-naphthyloxy)-propylamino]-buttersaeureamid
3-[2-Hydroxy-3-(1,2,3,4-tetrahydro-2-oxo-chinolin-5-yloxy)-propylamino]-N-(2-methyl-1-nitroxy-prop-2-yl)-propionsaeureamid
3-[2-Hydroxy-3-(1,2,3,4-tetrahydro-2-oxo-chinolin-5-yloxy)-propylamino]-N-(3-nitroxy-propyl)-propionsaeureamid
2-[3-(1,2-Dihydro-2-oxo-chinolin-5-yloxy)-2-hydroxy-propylamino]-2,N-dimethyl-N-(4-nitroxy-butyl)-propionsaeureamid
3-[3-(1,2-Dihydro-2-oxo-chinolin-5-yloxy)-2-hydroxy-propylamino]-3-methyl-N-(3-nitroxy-propyl)-buttersaeureamid
3-[3-(1,2-Dihydro-7-methyl-2-oxo-chinolin-5-yloxy)-2-hydroxy-propylamino]-N-(3-nitroxy-propyl)-propionsaeureamid
2-[3-(1,2-Dihydro-3-cyano-2-oxo-chinolin-5-yloxy)-2-hydroxy-propylamino]-propionsaeure-(4-nitroxymethyl)-piperidid
trans-4-[2-Hydroxy-3-(1,2,3,4-tetrahydro-2-formyl-isochinolin-5-yloxy)-propylamino]-4-methyl-N-(4-nitroxy-cyclohexyl)-valeriansaeureamid
3-[3-(2H-3,4-Dihydro-benzopyran-8-yloxy)-2-hydroxy-propylamino]-propionsaeure-(4-nitroxy)-piperidid
4-[3-(2H-3,4-Dihydro-benzopyran-8-yloxy)-2-hydroxy-propylamino]-4-methyl-N-(3-nitroxy-propyl)-valeriansaeureamid
trans-3-[2-Hydroxy-3-(2H-3-hydroxymethyl-benzopyran-8-yloxy)-propylamino]-3-methyl-N-(2-nitroxy-cyclohexyl)-buttersaeureamid
4-[3-(2H-3-Cyano-benzopyran-8-yloxy)-2-hydroxy-propylamino]-4-methyl-valeriansaeure-(4-nitroxymethyl)-piperidid
4-[2-Hydroxy-3-(2H-3-methoxycarbonyl-benzopyran-8-yloxy)-propylamino]-4-methyl-valeriansaeure-(4-nitroxymethyl)-piperidid
3-[3-(2H-3,4-Dihydro-2-oxo-benzopyran-8-yloxy)-2-hydroxy-propylamino]-3-methyl-buttersaeure(4-nitroxymethyl)-piperidid
trans-4-[2-Hydroxy-3-(2H-2-oxo-benzopyran-8-yloxy)-propylamino]-4-methyl-N-(2-nitroxy-cyclopentyl)-valeriansaeureamid
3-[2-Hydroxy-3-(2H-3-hydroxymethyl-2-oxo-benzopyran-8-yloxy)-propylamino]-3,N-dimethyl-N-(2-nitroxy-ethyl)-buttersaeureamid
3-[3-(2H-3,4-Dihydro-benzothiopyran-8-yloxy)-2-hydroxy-propylamino]-N-methyl-N-(4-nitroxy-butyl)-propionsaeureamid
4-[3-(2H-3,4-Dihydro-benzothiopyran-8-yloxy)-2-hydroxy-propylamino]-4-methyl-N-(3-nitroxy-propyl)-valeriansaeureamid
2-[3-(2,3-Dihydro-4H-3-oxo-1,4-benzoxazin-5-yloxy)-2-hydroxy-propylamino]-2-methyl-propionsaeure-(4-nitroxy)-piperidid
2-[3-(2,3-Dihydro-4H-3-oxo-1,4-benzoxazin-8-yloxy)-2-hydroxy-propylamino]-N-(3-nitroxy-propyl)-propionsaeureamid
cis-2-[3-(2,3-Dihydro-4H-3-oxo-1,4-benzothiazin-8-yloxy)-2-hydroxy-propylamino]-N-(4-nitroxy-cyclohexyl)-propionsaeureamid
3-[3-(2,3-Dihydro-1,4-benzodioxin-5-yloxy)-2-hydroxy-propylamino]-N-(2,2-dimethyl-3-nitroxy-propyl)-propionsaeureamid
4-[3-(2,3-Dihydro-1,4-benzodioxin-5-yloxy)-2-hydroxy-propylamino]-4-methyl-N-(3-nitroxy-propyl)-valeriansaeureamid
4-[3-(3,4-Dihydro-2,2-dioxo-1,2-benzoxathiin-8-yloxy)-2-hydroxy-propylamino]-buttersaeure-(4-nitroxymethyl)-piperidid
3-[3-(3,4-Dihydro-2,2-dioxo-1,2-benzoxathiin-8-yloxy)-2-hydroxy-propylamino]-3-methyl-N-(3-nitroxy-propyl)-buttersaeureamid
2-[3-(Carbazol-4-yloxy)-2-hydroxy-propylamino]-N-(1-nitroxy-but-3-yl)-essigsaeureamid
3-[3-Carbazol-4-yloxy)-2-hydroxy-propylamino]-3-methyl-buttersaeure-(4-nitroxy)-piperidid
trans-3-[2-Hydroxy-3-(2-hydroxymethyl-phenoxy)-propylamino]-3-methyl-N-[2-(2-nitroxy-cyclohexyl)-ethyl]-buttersaeureamid
2-[2-Hydroxy-3-(5-hydroxymethyl-2-methoxy-phenoxy)-propylamino]-2-methyl-N-(4-nitroxy-butyl)-propionsaeureamid
4-[2-Hydroxy-3-(5-hydroxymethyl-2-methtyl-phenoxy)-propylamino]-4-methyl-valeriansaeure-(4-nitroxymethyl)-piperidid
2-[3-(2,3-Dimethyl-phenoxy)-2-hydroxy-propylamino]-2-methyl-N-(4-nitroxy-butyl)-propionsaeureamid
trans-3-[3-(5-Chloro-2-methoxy-phenoxy)-2-hydroxy-propylamino]-3-methyl-N-[(2-nitroxy-cyclohexyl)-methyl]-buttersaeureamid
4-[2-Hydroxy-3-(2-methoxy-5-methyl-phenoxy)-propylamino]-4-methyl-valeriansaeure-(4-nitroxy)-piperidid

2-[2-Hydroxy-3-(2-methoxy-5-methyl-phenoxy)-propylamino]-2-methyl-propansulfonsaeure-(4-nitroxy)-piperidid
4-(2-Hydroxy-3-phenoxy-propylamino)-4-methyl-N-(3-nitroxy-propyl)-valeriansaeureamid
2-[2-Hydroxy-3-(3-methylphenoxy)-propylamino]-2-methyl-propionsäure-(4-nitroxy)-piperidid
2-[3-(2-Allyl-phenoxy)-2-hydroxy-propylamino]-2-methyl-propionsäure-(4-nitroxy)-piperidid
2-[3-(2-Cyano-phenoxy)-2-hydroxy-propylamino]-2-methyl-propionsäure-(4-nitroxy)-piperidid
2-[2-Hydroxy-3-(2-methoxy-phenoxy)-propylamino]-2-methyl-propionsäure-(4-nitroxy)-piperidid
2-[2-Hydroxy-3-(2-hydroxymethyl-indol-4-yloxy)-propylamino]-2-methyl-propionsäure-(4-nitroxy)-piperidid
2-[2-Hydroxy-3-(2-oxo-indolin-4-yloxy)-propylamino]-2-methyl-propionsäure-(4-nitroxy)-piperidid
2-[2-Hydroxy-3-(1-naphthyloxy)-propylamino]-2-methyl-propionsäure-(4-nitroxy)-piperidid
3-[2-Hydroxy-3-(2-oxo-indolin-4-yloxy)-propylamino]-propionsäure-(4-nitroxymethyl)-piperidid
3-[2-Hydroxy-3-(3-methyl-phenoxy)-propylamino]-3-methyl-buttersäure-(4-nitroxy)-piperidid
3-[3-(2-Allyl-phenoxy)-2-hydroxy-propylamino]-3-methyl-buttersäure-(4-nitroxy)-piperidid
3-[3-(2-Cyano-phenoxy)-2-hydroxy-propylamino]-3-methyl-buttersäure-(4-nitroxy)-piperidid
3-[2-Hydroxy-3-(2-methoxy-phenoxy)-propylamino]-3-methyl-buttersäure-(4-nitroxy)-piperidid
3-[2-Hydroxy-3-(indol-4-yloxy)-propylamino]-3-methyl-buttersäure-(4-nitroxy)-piperidid
3-[2-Hydroxy-3-(1-naphthyloxy)-propylamino]-3-methyl-buttersäure-(4-nitroxy)-piperidid
3-[2-Hydroxy-3-(3-methyl-phenoxy)-propylamino]-3-methyl-buttersäure-(4-nitroxymethyl)-piperidid
3-[3-(2-Allyl-phenoxy)-2-hydroxy-propylamino]-3-methyl-buttersäure-(4-nitroxymethyl)-piperidid
3-[3-(2-Cyano-phenoxy)-2-hydroxy-proyplamino]-3-methyl-buttersäure-(4-nitroxymethyl)-piperidid
3-[2-Hydroxy-3-(2-methoxy-phenoxy)-propylamino]-3-methyl-buttersäure-(4-nitroxymethyl)-piperidid
3-[2-Hydroxy-3-(indol-4-yloxy)-propylamino]-3-methyl-buttersäure-(4-nitroxymethyl)-piperidid
4-[3-(2-Allyl-phenoxy)-2-hydroxy-propylamino]-4-methyl-N-(2-nitroxy-ethyl)-valeriansäureamid
4-[2-Hydroxy-3-(indol-4-yloxy)-propylamino]-4-methyl-N-(2-nitroxy-ethyl)-valeriansäureamid
4-[2-Hydroxy-3-(indol-4-yloxy)-propylamino]-4-methyl-valeriansaeure-(4-nitroxy)-piperidid
4-[3-(2-Allyl-phenoxy)-2-hydroxy-propylamino]-4-methyl-N-[(1-ethoxycarbonyl-2-nitroxy)ethyl]-valeriansäureamid
4-[3-(2-Allyl-phenoxy)-2-hydroxy-propylamino-4-methyl-N-](1-carboxy-2-nitroxy)ethyl]-valeriansäureamid

Beispiel 1

3-[2-Hydroxy-3-(1-naphthyloxy)-propylamino]-3-methyl-N-(3-nitroxy-propyl)-buttersäureamid fumarat

21.0 g 3-Amino-3-methyl-N-(3-nitroxy-propyl)-buttersäureamid werden in 300 ml n-Butanol gelöst und mit 6.0g 2,3-Epoxy-1-(napthyl-1-oxy)-propan versetzt. Man rührt 15 h bei Raumtemperatur und engt im Vakuum ein. Der Rückstand wird in Ethylacetat gelöst, die organische Phase mehrmals mit Wasser gewaschen und über Natriumsulfat getrocknet. Nach Entfernen des Lösungsmittels wird an Kieselgel mit Methylenchlorid/Methanol (80:20) chromatographiert. Das erhaltene Öl (13.2 g) wird in Ethylacetat mit 3.68 g Fumarsäure versetzt. Der entstandene Niederschlag wird abgesaugt und getrocknet. Man erhält 10.8 g (68% d.Th.) Fumarat vom Schmelzpunkt 120-122°C.

In analoger Weise wurden erhalten:

| Bezeichnung | | Ausb. % | Schmelzpunkt °C (Lsg.mittel) |
|---|---|---|---|
| 1) | 3-[2-Hydroxy-3-(3-methyl-phenoxy)-propylamino]-3-methyl-N-(3-nitroxy-propyl)-buttersäureamid fumarat aus 2,3-Epoxy-1-(3-methyl-phenoxy)-propan und 3-Amino-3-methyl-N-(3-nitroxy-propyl)-buttersäure-amid | 21 | 127-128 (Ethylacetat) |
| 2) | 3-[3-(2-Allyl-phenoxy)-2-hydroxy-propylamino]-3-methyl-N-(3-nitroxypropyl)-buttersäureamid aus 1-(2-Allyl-phenoxy)-2,3-epoxy-propan und 3-Amino-3-methyl-N-(3-nitroxy-propyl)-buttersäure-amid | 52 | Oel |
| 3) | 3-[3-(2-Cyano-phenoxy)-2-hydroxy-propylamino]-3-methyl-N-(3-nitroxy-propyl)-buttersäureamid aus 1-(2-Cyano-phenoxy)-2,3-epoxy-propan und 3-Amino-3-methyl-N-(3-nitroxy-propyl)-buttersäure-amid | 32 | Oel |
| 4) | 3-[2-Hydroxy-3-(2-methoxy-phenoxy)-propylamino]-3-methyl-N-(3-nitroxy-propyl)-buttersäureamid fumarat aus 2,3-Epoxy-1-(2-methoxy-phenoxy)-propan und 3-Amino-3-methyl-N-(3-nitroxy-propyl)-buttersäure-amid | 16 | 95-98 (Ethylacetat) |

| | Bezeichnung | Ausb. % | Schmelzpunkt °C (Lsg.mittel) |
|---|---|---|---|
| 5) | 3- [2-Hydroxy-3-(4-methylcarbonyloxy-2,3,5-trimethylphenoxy)-propylamino]-3-methyl-N-(3-nitroxy-propyl)-buttersäureamid fumarat<br><br>aus<br>2,3-Epoxy-1-(4-methylcarbonyloxy-2,3,5-trimethyl-phenoxy)-propan<br>und<br>3-Amino-3-methyl-N-(3-nitroxy-propyl)-buttersäureamid | 42 | 104-106<br>(Ethylacetat) |
| 6) | 4-[3-(2-Allyl-phenoxy)-2-hydroxy-propylamino]-4-methyl-valeriansäure-(4-nitroxy)-piperidid fumarat<br><br>aus<br>1-(2-Allyl-phenoxy)-2,3-epoxy-propan<br>und<br>4-Amino-4-methyl-valeriansäure-(4-nitroxy)-piperidid | 14 | 112-114<br>(Ethylacetat) |
| 7) | 2-[2-Hydroxy-3-(2-methoxyphenoxy)-propylamino-]2-methyl-N-(3-nitroxy-propyl)-propionsäureamid cyclaminat<br><br>aus<br>2,3-Epoxy-1-(2-methoxyphenoxy)-propan<br>und<br>2-Amino-2-methyl-N-(3-nitroxy-propyl)-propionsäureamid | 39 | 133-135<br>(Ethylacetat) |
| 8) | 2-[2-Hydroxy-3-(3-methylphenoxy)-propylamino]-2-methyl-N-(3-nitroxy-propyl)-propionsäureamid cyclaminat<br><br>aus<br>2,3-Epoxy-1-(3-methylphenoxy)-propan<br>und<br>2-Amino-2-methyl-N-(3-nitroxy-propyl)-propionsäureamid | 48 | 134-136<br>(Ethylacetat) |

| | Bezeichnung | Ausb. % | Schmelzpunkt °C (Lsg.mittel) |
|---|---|---|---|
| 9) | 2-[3-(2-Allyl-phenoxy)-2-hydroxy-propylamino]-2-methyl-N-(3-nitroxy-propyl)-propionsäureamid cyclaminat<br><br>aus<br>1-(2-Allyl-phenoxy)-2,3-epoxy-propan<br>und<br>2-Amino-2-methyl-N-(3-nitroxy-propyl)-propionsäure-amid | 36 | 128-129 (Ethylacetat) |
| 10) | 2-[3-(2-Cyano-phenoxy)-2-hydroxy-propylamino]-2-methyl-N-(3-nitroxy-propyl)-propionsäureamid cyclaminat<br><br>aus<br>1-(2-Cyano-phenoxy)-2,3-epoxy-propan<br>und<br>2-Amino-2-methyl-N-(3-nitroxy-propyl)-propion-säureamid | 37 | 103-105 (Ethylacetat) |
| 11) | 2-[2-Hydroxy-3-(2-methyl-phenoxy)-propylamino]-2-methyl-N-(3-nitroxy-propyl)-propionsäureamid cyclaminat<br><br>aus<br>2,3-Epoxy-1-1(2-methyl-phen-oxy)-propan<br>und<br>2-Amino-2-methyl-N-(3-nitroxy-propyl)-propion-säureamid | 48 | 148-150 (Ethylacetat) |

| | Bezeichnung | Ausb. % | Schmelzpunkt °C (Lsg.mittel) |
|---|---|---|---|
| 12) | 4-[2-Hydroxy-3-(3-methyl-phenoxy)-propylamino]-4-methyl-N-(3-nitroxy-propyl)-valeriansäureamid benzoat<br><br>aus<br>2,3-Epoxy-3-(3-methyl-phenoxy)-propan<br>und<br>4-Amino-4-methyl-N-(3-nitroxy-propyl)-valeriansäureamid | 34 | 104-107 (Aceton) |
| 13) | 4-[3-(2-Allyl-phenoxy)-2-hydroxy-propylamino]-4-methyl-N-(3-nitroxy-propyl)-valeriansäureamid<br><br>aus<br>1-(2-Allyl-phenoxy)-2,3-epoxy-propan<br>und<br>4-Amino-4-methyl-N-(3-nitroxy-propyl)-valeriansäureamid | 59 | Öl |
| 14) | 4-[3-(2-Allyl-phenoxy)-2-hydroxy-propylamino]-4-methyl-valeriansäure-(4-nitroxymethyl)-piperidid fumarat<br>aus<br>1-(2-Allyl-phenoxy)-2,3-epoxy-propan<br>und<br>4-Amino-4-methyl-valeriansäure-(4-nitroxymethyl)-piperidid | 54 | 135-138 (Ethylacetat) |

| | Bezeichnung | Ausb. % | Schmelzpunkt °C (Lsg.mittel) |
|---|---|---|---|
| 15) | 4-[3-(2-Cyano-phenoxy)-2-hydroxy-propylamino]-4-methyl-N-(3-nitroxy-proypl)-valeriansäureamid benzoat<br><br>aus<br>1-(2-Cyano-phenoxy)-2,3-epoxy-propan<br>und<br>4-Amino-4-methyl-N-(3-nitroxy-propyl)-valeriansäureamid | 15 | 128-130 (Aceton) |
| 16) | 4-[2-Hydroxy-3-(2-methoxy-phenoxy)-propylamino]-4-methyl-N-(3-nitroxy-propyl)-valeriansäureamid benzoat<br><br>aus<br>2,3-Epoxy-1-(2-methoxy-phenoxy)-propan<br>und<br>4-Amino-4-methyl-N-(3-nitroxy-propyl)-valeriansäureamid | 28 | 102-104 (Aceton) |
| 17) | 4-[2-Hydroxy-3-(1-naphthyl-oxy)-propylamino]-4-methyl-N-(3-nitroxy-propyl)-valeriansäureamid fumarat<br><br>aus<br>2,3-Epoxy-1-(1-napthyloxy)-propan<br>und<br>4-Amino-4-methyl-N-(3-nitroxy-propyl)-valeriansäureamid | 64 | 134-136 (Ethylacetat) |

Beispiel 2

3-[2-Hydroxy-3-(indol-4-yloxy)-propylamino]-N-(3-nitroxy-propyl)-propionsäureamid cyclaminat

3.8 g (0.02 mol) 2,3-Epoxy-1-(indol-4-yloxy)-propan werden in 100 ml Ethanol gelöst und mit 21.3 g (0.1 mol) 3-Amino-N-(3-nitroxy-propyl)-propionsäureamid versetzt. Nach 2 d Rühren bei Raumtemperatur wird im Vaccum bei max. 20°C Badtemperatur eingeengt. Der Rückstand wird in 200 ml Ethylacetat gelöst und 3 x mit 100 ml Wasser extrahiert. Die Ethylacetatphase wird darauf mit 2 N Milchsäure extrahiert, dann durch Zugabe von Kaliumcarbonat die Base wieder freigesetzt und mit Essigester extrahiert. Die organische Phase wird mit $Na_2SO_4$ getrocknet und im Vacuum bei 20°C abdestilliert. Es verbleiben 5.8 g Öl. Durch Lösen in 50 ml Ethanol und Zugabe von 2.7 g Cyclohexansulfaminsäure werden nach Absaugen der Kristalle 2.2 g Cyclaminat der Titelverbindung vom Schmp. 124-125°C erhalten, d.s. 20 % d.Th..

In analoger Weise werden erhalten:

| | Bezeichnung | Ausb. % | Schmelzpunkt °C (Lsg.mittel) |
|---|---|---|---|
| 1) | 3-[2-Hydroxy-3-(2-hydroxymethyl-indol-4-yloxy)-propylamino]-N-(3-nitroxy-propyl)-propionsäureamid cyclaminat<br>aus<br>2,3-Epoxy-1-(2-hydroxy-methyl-indol-4-yloxy)-propan<br>und<br>3-Amino-N-(3-nitroxy-propyl)-propionsäureamid | 20 | 108-109 (Ethanol/Diethylether) |
| 2) | 2-[2-Hydroxy-3-(indol-4-yloxy)-propylamino]-2-methyl-N-(3-nitroxy-propyl)-propionsäureamid cyclaminat<br>aus<br>2,3-Epoxy-1-(indol-4-yloxy)-propan<br>und<br>2-Amino-2-methyl-N-(3-nitroxy-propyl)-propionsäureamid- | 40 | 131-132 (Ethanol) |
| 3) | 2-[2-Hydroxy-3-(2-hydroxymethyl-indol-4-yloxy)-propylamino]-2-methyl-N-(3-nitroxy-propyl)-propionsäureamid cyclaminat<br>aus<br>2,3-Epoxy-1-(2-hydroxy-methyl-indol-4-yloxy)-propan<br>und<br>2-Amino-2-methyl-N-(3-nitroxy-propyl)-propionsäureamid- | 42 | 128 (Ethanol) |
| 4) | 2-[2-Hydroxy-3-(2-oxo-indolin-4-yloxy)-propylamino]-2-methyl-N-(3-nitroxy-propyl)-propionsäureamid cyclaminat<br>aus<br>2,3-Epoxy-1-(2-oxo-indolin-4-yloxy)-propan<br>und<br>2-Amino-2-methyl-N-(3-nitroxy-propyl)-propionsäureamid- | 20 | 158-160 (Ethanol) |

| | Bezeichnung | Ausb. % | Schmelzpunkt °C (Lsg.mittel) |
|---|---|---|---|
| 5) | 3-[2-Hydroxy-3-(2-hydroxy-methyl-indol-4-yloxy)-propyl-amino]-propionsäure-(4-nitroxy)-piperidid cyclaminat aus 2,3-Epoxy-1-(2-hydroxy-methyl-indol-4-yloxy)-propan und 3-Amino-propionsäure-(4-nitroxy)-piperidid | 20 | 131 (Ethanol) |
| 6) | 3-[2-Hydroxy-3-(indol-4-yl-oxy)-propylamino]-propion-säure-(4-nitroxy)-piperidid hemifumarat aus 2,3-Epoxy-1-(indol-4-yloxy)-propan und 3-Amino-propionsäure-(4-nitroxy)-piperidid | 15 | 177-180 (Ethanol) |
| 7) | 3-[2-Hydroxy-3-(2-hydroxy-methyl-indol-4-yloxy)-prop-ylamino]-propionsäure-(4-nitroxy)-piperidid cyclaminat aus 2,3-Epoxy-1-(2-hydroxy-methyl-indol-4-yloxy)-propan und 3-Amino-propionsäure-(4-nitroxy)-piperidid | 10 | 131 (Ethanol) |
| 8) | 2-[2-Hydroxy-3-(indol-4-yl-oxy)-propylamino]-2-methyl-propionsäure-(4-nitroxy)-piperidid hemifumarat aus 2,3-Epoxy-1-(indol-4-yloxy)-propan und 2-Amino-2-methyl-propion-säure-(4-nitroxy)-piperidid | 50 | 171-172 (Ethanol) |

| | Bezeichnung | Ausb. % | Schmelzpunkt °C (Lsg.mittel) |
|---|---|---|---|
| 9) | 4-[2-Hydroxy-3-(indol-4-yl-oxy)-propylamino]-4-methyl-N-(3-nitroxy-propyl)-valeriansäureamid hemifumarat aus 2,3-Epoxy-1-(indol-4-yloxy)-propan und 4-Amino-4-methyl-N-(3-nitroxy-propyl)-valerian-säureamid | 43 | 75 (Ethanol) |
| 10) | 4-[2-Hydroxy-3-(2-oxo-indolin-4-yloxy)-propyl-amino]-4-methyl-valerian-säure-(4-nitroxy)-piperidid hemifumarat aus 2,3-Epoxy-1-(2-oxo-indolin-4-yloxy)-propan und 4-Amino-4-methyl-valerian-säure-(4-nitroxy)-piperidid | 10 | 166 (Ethnaol) |
| 11) | 4-[2-Hydroxy-3-(indol-4-yl-oxy)-propylamino-4-methyl-valeriansäure-(4-nitroxy-methyl)-piperidid hemifumarat aus 2,3-Epoxy-1-(indol-4-yloxy)-propan und 4-Amino-4-methyl-valerian-säure-(4-nitroxymethyl)-piperidid | 30 | 130 (Ethanol) |

Beispiel 3

3-[2-Hydroxy-3-(4-oxo-indolin-4-yloxy)-propylamino]-N-(3-nitroxy-propyl)-propionsäureamid cyclaminat

3.5 g (0.017 mol) 2,3-Epoxy-1-(2-oxo-indolin-4-yloxy)-propan werden in einer Mischung aus 50 ml Methylenchlorid und 50 ml Ethanol gelöst und mit 11 g (0.05 mol) 3-Amino-N-(3-nitroxy-propyl)-propionsäureamid versetzt. Nach 24 h Rühren bei Raumtemperatur wird im Vaccum das Methylenchlorid abdestilliert und die verbleibende alkoholische Lösung noch 24 h bei Raumtemperatur gerührt. Die gebildeten Kristalle werden abgesaugt und mit Ethanol und Ether nachgewaschen. Es werden 3.9 g Rohbase erhalten. Nach Anschlämmen in 20 ml Ethanol und Zugabe von 1.8 g Cyclohexylsulfaminsäure wird eine Lösung erhalten, aus welcher das Salz auskristallisiert. Es werden 4.9 g Cyclaminat der Titelverbindung vom Schmp. 160-164°C erhalten, d.s. 50 % d.Th..

Beispiel 4

3-Amino-N-(3-nitroxy-propyl)-propionsäureamid fumarat

28.7 g (0.15 mol) 3-Amino-N-(3-hydroxy-propyl)-propionsäureamid hemioxalat werden unter Rühren bei 5°C in 63 ml 100proz. Salpetersäure eingetragen. Man läßt die Reaktionslösung noch etwa 1 h im Eisbad bei +5°C rühren, gibt dann 1 L Methylenchlorid zu und neutralisiert unter weiterem Rühren mit 2.34 g Kaliumcarbonat x 1.5 $H_2O$. Nach einstündigem Rühren im Eisbad werden 100 ml Ethanol zugesetzt und noch etwa 18 h bei Raumtemperatur gerührt. Die Suspension wird abgesaugt und das Filtrat im Vacuum bei max 20°C abdestilliert. Es verbleiben etwa 32 g Rohbase. Durch Lösen in Ethanol und Zugabe von 17.4 g Fumarsäure erhält man 23 g Fumarat der Titelverbindung vom Schmp. 119 - 120° C d. s. 50 % d. Th.

In analoger Weise werden erhalten:

| Bezeichnung | | Ausb. % | Schmelzpunkt °C (Lsg.mittel) |
|---|---|---|---|
| 1) | 3-Amino-3-methyl-N-(3-nitroxypropyl)-buttersäure-amid aus 3-Amino-3-methyl-N-(3-hydroxypropyl)-buttersäureamid oxalat | 90 | Öl |
| 2) | 2-Amino-2-methyl-N-(3-nitroxypropyl)-propionsäureamid fumarat aus 2-Amino-2-methyl-N-(3-hydroxypropyl)-propionsäureamid hemioxalat | 21 | 133-134 (Ethanol) |
| 3) | 4-Amino-4-methyl-valeriansäure-(4-nitroxy)-piperidid fumarat aus 4-Amino-4-methyl-valeriansäure-(4-hydroxy)-piperidid oxalat | 29 | 263-265 (Ethylacetat) |
| 4) | 3-Amino-propionsäure-(4-nitroxy)-piperidid fumarat aus 3-Amino-propionsäure-(4-hydroxy)-piperidid | 64 | 141-142 (Ethanol) |

| | Bezeichnung | Ausb. % | Schmelzpunkt °C (Lsg.mittel) |
|---|---|---|---|
| 5) | 4-Amino-4-methyl-N-(3-nitroxy-propyl)-valeriansäureamid fumarat<br><br>aus<br>4-Amino-4-methyl-N-(3-hydroxy-propyl)-valeriansäureamid oxalat | 46 | 102<br>(Ethylacetat/Aceton) |
| 6) | 4-Amino-4-methyl-valeriansäure-(4-nitroxymethyl)-piperidid fumarat<br><br>aus<br>4-Amino-4-methyl-valeriansäure-(4-hydroxymethyl)-piperidid oxalat | 65 | 142-145<br>(Ethylacetat/Aceton) |
| 7) | 2-Amino-2-methyl-propionsäure-(4-nitroxy)-piperidid fumarat<br>aus<br>2-Amino-2-methyl-propionsäure-(4-hydroxy)-piperidid hemioxalat | 50 | 185<br>(Ethanol) |

Beispiel 5

3-Amino-N-(3-hydroxy-propyl)-propionsäureamid hemioxalat

113 g 2-Cyanessigsäureethylester werden in 1 l Methylenchlorid gelöst und mit 75.1 g 3-Amino-1-propanol versetzt. Nach 3 d rühren bei Raumtemperatur wird im Vacuum abdestilliert. Es verbleiben 153 g Rohprodukt von 2-Cyan-N-(3-hydroxy-propyl)-essigsäureamid, d.s. ca. 100 % d.Th..

61 g dieses Produkts werden in 300 ml Methanol gelöst, mit 300 ml flüssigem Ammoniak versetzt und mit 20 g Raney-Nickel bei 100 b Wasserstoffdruck und Raumtemperatur hydriert. Nach Absaugen des Katalysators wird das Lösungsmittel im Vacuum abdestilliert, der Rückstand in 400 ml Ethanol gelöst und mit 19.4 g Oxalsäure in 200 ml Ethanol versetzt. Nach Stehen über Nacht wird abgesaugt. Es verbleiben 64.2 g Hemioxalat der Titelverbindung vom Schmp. 140-142°C, d.s. 78 % d.Th..

Analog wird erhalten:

| Bezeichnung | | Ausb. % | Schmelzpunkt °C (Lsg.mittel) |
|---|---|---|---|
| 1) | 3-Amino-propionsäure-(4-hydroxy)-piperidid hemioxalat aus 2-Cyanessigsäureethylester 4-Hydroxy-piperidin | 75 | 170-172 (Ethanol) |

Beispiel 6

2-Amino-2-methyl-N-(3-hydroxy-propyl)-propionsäureamid

30 g 3-Amino-1-propanol werden mit 129 g 2-Methyl-2-nitro-propionsäuremethylester versetzt und 8 h im Ölbad auf 120°C erhitzt. Man destilliert im Vacuum bei 15 Torr. den Überschuß an Ester ab. Es verbleiben im Rückstand 76 g Rohprodukt von 2-Methyl-2-nitro-N-(3-hydroxy-propyl)-propionsäureamid.

Dieses wird ohne weitere Reinigung in 1 L Methanol gelöst und bei Raumtemperatur und 1 bar Wasserstoffdruck über Raney-Nickel hydriert. Man saugt vom Katalysator ab und destilliert im Vacuum das Lösungsmittel ab. Es verbleiben etwa 65 g Rohbase. Diese wird in 650 ml Ethanol gelöst und mit 18 g wasserfreier Oxalsäure versetzt. Nach Stehen über Nacht wird abgesaugt. Man erhält 70 g Hemioxalat der Titelverbindung vom Schmp. 156-158°C, d.s. 85 % d.Th.

Analog wird erhalten:

| | Bezeichnung | Ausb. % | Schmelzpunkt °C (Lsg.mittel) |
|---|---|---|---|
| 1) | 2-Amino-2-methyl-propionsäure-(4-hydroxy)-piperidid hemioxalat aus 2-Methyl-2-nitro-propionsäuremethylester und 4-Hydroxy-piperidin | 30 | 197 (Ethanol) |

Beispiel 7

3-Amino-3-methyl-N-(3-hydroxy-propyl)-buttersäureamid hemioxalat

100 ml 3,3-Dimethyl-acrylsäureethylester werden in 1.5 L Ethanol gelöst und im Autoklaven mit 1.5 L Ammoniak versetzt. Man presst Stickstoff auf (100 b) und erwärmt 110 h auf 50°C. Nach Entspannen und Entfernen des Lösungsmittels verbleiben 90 g (86 % d.Th.) Rohprodukt von 3-Amino-3-methyl-buttersäureethylester.

33.6 g dieses Produkt werden in 110 ml Ethanol gelöst, in einen Autoklaven gegeben und mit 87 ml 3-Hydroxy-propylamin versetzt. Man rührt 100 h bei 50°C unter einer Stickstoffatmosphäre von 100 b. Nach dem Entspannen und dem Entfernen des Lösungsmittels wird im Glasrohrofen destilliert. Man erhält bei 0.04 b und 190°C Ofentemperatur 26.5 g (66 % d.Th.) Öl von 3-Amino-3-methyl-N-(3-hydroxy-propyl)-buttersäureamid. Dieses wird in Isopropanol gelöst und heiß mit 6.84 g Oxalsäure versetzt. Man läßt abkühlen und saugt ab. Man erhält 27.0 g (53 % d.Th.) Kristalle der Titelverbindung vom Schmelzpunkt 123-125°C.

Analog wird erhalten.

| | Bezeichnung | Ausb. % | Schmelzpunkt °C (Lsg.mittel) |
|---|---|---|---|
| 1) | 3-Amino-3-methyl-N-(2-hydroxy-ethyl)-buttersäure-amid aus 3-Amino-3-methyl-butter-säureethylester und 2-Hydroxy-ethylamin | 44 | Öl |

Beispiel 8

4-Amino-4-methyl-valeriansäure-(4-hydroxy)-piperidid oxalat

Zu einer Lösung von 180 ml 2-Nitropropan in 25.8 ml 35proz. Benzyltrimethylammoniumhydroxid-Lösung und 100 ml t-Butanol tropft man bei ca. 35°C 181 ml Acrylsäuremethylester. Man läßt abkühlen und extrahiert mit Methylenchlorid und Wasser, trocknet die organische Phase mit Natriumsulfat und engt ein. Der Rückstand wird fraktioniert destilliert ($Kp_3$ = 84-87°C). Man erhält 285 g (82 % d.Th.) 4-Methyl-4-nitro-valeriansäuremethylester.

175 g dieses Produkts werden mit 500 ml 2 N-Natronlauge versetzt und 4 h bei Raumtemperatur gerührt. Das Wasser wird abdestilliert und der feste Rückstand im Vacuumtrockenschrank getrocknet. Man erhält 183.1 g (100 % d.Th.) Natriumsalz von 4-Methyl-4-nitro-valeriansäure.

Zu einer Suspension dieses Salzes in 1 L Methylenchlorid gibt man unter Kühlung 208.2 g Phosphorpentachlorid. Man läßt 15 h bei Raumtemperatur rühren, saugt vom Ungelösten ab und engt das Filtrat ein. Es verbleiben 153 g (85 % d. Th.) eines Öls von 4-Methyl-4-nitro-valeriansäurechlorid.

Zu einer Lösung von 4-Hydroxy-piperidin in 340 ml Aceton, 35.7 g Natriumacetat und 218 ml Wasser tropft man bei 0-5°C eine Lösung von 39.1 g 4-Methyl-4-nitro-valeriansäurechlorid in 108 ml Aceton. Man läßt 15 h bei Raumtemperatur rühren, destilliert das Lösungsmittel ab, nimmt den Rückstand mit 600 ml Methylenchlorid auf und wäscht zweimal mit je 30 ml gesättigter Natriumchlorid-Lösung. Die organische Phase wird getrocknet und eingeengt. Es verbleiben 57.9 g des Rohprodukts von 4-Methyl-4-nitro-valeriansäure-(4-hydroxy)-piperidid.

Obiges Rohprodukt wird in 600 ml Ethanol gelöst, im Autoklaven mit 600 ml Ammoniak und 8 g Raney-Nickel versetzt und 10 h bei 40°C und 80 b Wasserstoffdruck hydriert. Man entspannt, engt ein und verreibt den festen Rückstand mit Ethylacetat. Es verbleiben 33.8 g eines Feststoffes. Dieses wird in Isopropanol gelöst und in der Hitze mit 14.2 g Oxalsäure versetzt. Man läßt abkühlen und saugt ab. Man erhält 39.5 g (55 %) der Titelverbindung vom Schmelzpunkt 106-109°C.

Analog werden erhalten:

| | Bezeichnung | Ausb. % | Schmelzpunkt °C (Lsg.mittel) |
|---|---|---|---|
| 1) | 4-Amino-4-methyl-N-(3-hydroxy-propyl)-valeriansäureamid hemioxalat<br><br>aus<br>4-Methyl-4-nitro-valeriansäurechlorid<br>und<br>3-Hydroxy-propylamin | 80 | 166<br>(Ethanol) |

| | Bezeichnung | Ausb. % | Schmelzpunkt °C (Lsg.mittel) |
|---|---|---|---|
| 2) | 4-Amino-4-methyl-N-(2-hydroxy-ethyl)-valeriansäureamid hemioxalat<br><br>aus<br>4-Methyl-4-nitro-valeriansäurechlorid<br>und<br>2-Hydroxy-ethylamin | 64 | 91<br>(Ethanol) |
| 3) | 4-Amino-4-methyl-valeriansäure-(4-hydroxymethyl)-piperidid oxalat<br><br>aus<br>4-Methyl-4-nitro-valeriansäurechlorid<br>und<br>4-Hydroxymethyl-piperidin | 36 | Öl |

**Ansprüche**

1. Verbindungen der allgemeinen Formel I

$$Ar-O-CH_2-\overset{OH}{\overset{|}{C}}H-CH_2-NH-A-X-\underset{R}{\underset{|}{N}}-B-ONO_2 \qquad (I)$$

worin

Ar eine substituierte oder unsubstituierte aromatische oder heteroaromatische Gruppe bedeutet,

A eine geradkettige oder verzweigte Alkylenkette von 1-8 C-Atomen, wobei eine $-CH_2$-Gruppe durch eine Cycloalkylengruppe von 3-7 C-Atomen ersetzt sein kann, bedeutet

X eine -C(=O)-, -S(=O)-oder $-S(=O)_2$-Gruppe bedeutet

B eine geradkettige, mono-oder bicyclische, gegebenenfalls verzweigte, gesaettigte oder ungesaettigte Alkylenkette von 1-12 C-Atomen, worin eine $-CH_2$-Gruppe durch eine Cycloalkylen-Gruppe von 3-7 C-Atomen ersetzt sein kann und/oder bis zu 2 $-CH_2$-Gruppen jeweils durch ein Sauerstoffatom oder Schwefelatom oder eine -S(=O)-oder $-S(=O)_2$-Gruppe ersetzt sein koennen, bedeutet

R Wasserstoff oder eine geradkettige oder verzweigte, gesaettigte oder ungesaettigte Alkyl-oder Nitroxyalkyl-Gruppe von 1-6 C-Atomen darstellt, oder R auch gemeinsam mit dem benachbarten Stickstoffa-

tom und einer $-CH_2$-Gruppe der Kette B einen heteroaliphatischen Ring mit 4-6 C-Atomen aufspannen kann, bedeutet
sowie deren physiologisch vertraegliche Salze.

2. Verbindungen der allgemeinen Formel I gemäß Anspruch 1, in der
Ar eine unsubstituierte oder durch Halogen-, Cyano-, Hydroxy-, Amino-, Formyl-, Nitro-, Carboxyl-, Carbamoyl-, Trifluormethyl-, Alkyl-, Alkenyl-, Alkinyl-, Cycloalkyl-, Bicycloalkyl-, Alkanoyl-, Alkoxy-, Hydroxyalkyl-, Alkoxyalkyl-, Alkoxyalkoxyalkyl-, Alkoxyalkoxy-, Alkenyloxy-, Alkenyloxyalkyl-, Alkinyloxy-, Alkinyloxyalkyl-, Cycloalkoxy-, Alkylthio-, Alkylthioalkyl-, Morpholino-, Acylamino-, Acylaminoalkyl-, Acyloxy-, Alkoxycarbonyl-, Cycloalkyloxycarbonyl-, Alkylaminocarbonylamino-, Dialkylaminocarbonylamino-, Dialkylaminocarbonylaminoalkyl-, Dialkylaminocarbonylalkyl-, Dialkylaminocarbonylalkoxy-, wobei bei den letzteren 4 Gruppen die beiden endstaendigen Alkylgruppen zusammen mit dem Stickstoffatom auch eine cyclische Gruppe mit vier oder fuenf C-Atomen bilden koennen, Cycloalkylaminocarbonylamino-, Alkylaminocarbonylaminoalkyl-, Cycloalkylaminocarbonylaminoalkyl-, Alkoxycarbonylaminoalkyl-, Cycloalkoxycarbonylaminoalkyl-, Carbamoylalkyl-, Alkylaminocarbonylalkyl-, Cycloalkylamincarbonylalkyl oder Alkylaminocarbonylalkoxy-Gruppen substituierte Phenylgruppe
oder eine unsubstituierte oder durch Alkyl-, Cyano-, Hydroxyalkyl-, Hydroxy-, Oxo-, Formyl-, Alkanoyl-, Alkylcarbonylamino-, Alkoxycarbonyl-oder Morpholino-gruppen substituierte Thiadiazolyl-, Naphthyl-, Indenyl-, Indolyl-, Indazolyl-, Imidazolyl-, Benzimidazolyl-, Benztriazolyl-, Benzofuranyl-, Benzodioxolyl-, Benzothiophenyl-, Benzthiazolyl-, Benzisothiazolyl-, Chinolyl-, Isochinolyl-, Benzodiazinyl-, Benzopyranyl-, Benzothiinyl-, Benzothiazinyl-, Benzothiadiazinyl-, Benzoxathiinyl-, Benzoxazolyl-, Benzisoxazolyl-, Benzoxazinyl-, Benzodioxinyl-oder Carbazolyl-Gruppe, wobei eine oder mehrere Doppelbindungen hydriert sein können, bedeutet,
A eine geradkettige oder verzweigte Alkylenkette von 1-8 C-Atomen, wobei eine $-CH_2$-Gruppe durch eine Cycloalkylengruppe von 3-7 C-Atomen ersetzt sein kann, bedeutet
X eine -C(=O)-, -S(=O)-oder $-S(=O)_2$-Gruppe bedeutet
B eine geradkettige, mono-oder bicyclische, gegebenenfalls verzweigte, gesaettigte oder ungesaettigte Alkylenkette von 1-12 C-Atomen, worin eine $-CH_2$-Gruppe durch eine Cycloalkylen-Gruppe von 3-7 C-Atomen ersetzt sein kann und/oder bis zu 2 $-CH_2$-Gruppen jeweils durch ein Sauerstoffatom oder Schwefelatom oder eine -S(=O)-oder $-S(=O)_2$-Gruppe ersetzt sein koennen, bedeutet
R Wasserstoff oder eine geradkettige oder verzweigte, gesaettigte oder ungesaettigte Alkyl-oder Nitroxyalkyl-Gruppe von 1-6 C-Atomen darstellt, oder R auch gemeinsam mit dem benachbarten Stickstoffatom und einer $-CH_2$-Gruppe der Kette B einen heteroaliphatischen Ring mit 4-6 C-Atomen aufspannen kann, bedeutet
sowie deren physiologisch vertraegliche Salze.

3. Verbindungen der allgemeinen Formel I gemäß Anspruch 1 oder 2, in der
A eine geradkettige Alkylenkette mit 1 - 3 C-Atomen oder eine verzweigte Alkylenkette mit 2 - 5 C-Atomen bedeutet,
X eine -C(=O)-, -S(=O)-oder $-S(=O)_2$-Gruppe bedeutet,
B eine geradkettige oder verzweigte Alkylenkette mit 2 - 6 C-Atomen bedeutet,
R Wasserstoff oder einen geradkettigen oder verzweigten Alkylrest mit 1 - 6 C-Atomen oder R gemeinsam mit dem benachbarten Stickstoffatom und einer $CH_2$-Gruppe der Kette B einen heteroaliphatischen Ring mit 4 - 6 C-Atomen aufspannen kann, bedeutet,
sowie deren physiologisch verträgliche Salze.

4. Verfahren zur Herstellung von Verbindungen der allgemeinen Formel I

$$\begin{array}{c} \text{OH} \\ | \\ \text{Ar-O-CH}_2\text{-CH-CH}_2\text{-NH-A-X-}\underset{\displaystyle \overset{|}{\text{R}}}{\text{N}}\text{-B-ONO}_2 \end{array} \qquad (I)$$

worin
Ar eine substituierte oder unsubstituierte aromatische oder heteroaromatische Gruppe bedeutet,
A eine geradkettige oder verzweigte Alkylenkette von 1-8 C-Atomen, wobei eine $-CH_2$-Gruppe durch eine Cycloalkylengruppe von 3-7 C-Atomen ersetzt sein kann, bedeutet
X eine -C(=O)-, -S(=O)-oder $-S(=O)_2$-Gruppe bedeutet
B eine geradkettige, mono-oder bicyclische, gegebenenfalls verzweigte, gesaettigte oder ungesaettigte Alkylenkette von 1-12 C-Atomen, worin eine $-CH_2$-Gruppe durch eine Cycloalkylen-Gruppe von 3-7 C-Atomen ersetzt sein kann und/oder bis zu 2 $-CH_2$-Gruppen jeweils durch ein Sauerstoffatom oder Schwefelatom oder eine $-S(=O)$-oder $-S(=O)_2$-Gruppe ersetzt sein koennen, bedeutet
R Wasserstoff oder eine geradkettige oder verzweigte, gesaettigte oder ungesaettigte Alkyl-oder Nitroxyalkyl-Gruppe von 1-6 C-Atomen darstellt, oder R auch gemeinsam mit dem benachbarten Stickstoffatom und einer $-CH_2$-Gruppe der Kette B einen heteroaliphatischen Ring mit 4-6 C-Atomen aufspannen kann, bedeutet,
dadurch gekennzeichnet, daß man in an sich bekannter Weise
a) eine Verbindung der allgemeinen Formel II

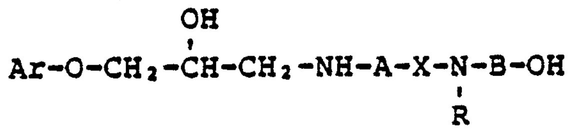 (II)

in der A, Ar, B, X und R die oben angegebenen Bedeutungen besitzen, einer Nitratester-Bildungsreaktion unterwirft, oder
b) eine Verbindung der allgemeinen Formel III
Ar'-O-Z (III)
in der Ar' die gleiche Bedeutung wie Ar hat oder gegebenenfalls eine entsprechende Synthesevorstufe sein kann und Z eine der Gruppierungen

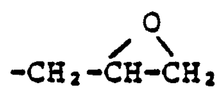 oder 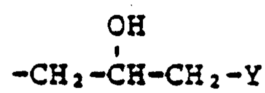

darstellt, worin Y eine reaktive Gruppe bedeutet,
b1) mit einem Amin der allgemeinen Formel IV
$H_2N$-A-X- N -B-$ONO_2$ (IV)
R
in der A, B, X und R die oben angegebenen Bedeutungen haben, oder
b2) mit einem Amin der allgemeinen Formel V
$H_2N$-A-X-U (V)
worin A und X die oben angegebenen Bedeutungen haben und U eine nucleofuge Gruppe ist
zu einer Verbindung der allgemeinen Formel VI
OH
Ar'-O-$CH_2$- CH -$CH_2$-NH-A-X-U (VI)
umsetzt und diese, gegebenenfalls nach Ueberfuehrung der Gruppe Ar' in die Gruppe Ar mit einer Verbindung der allgemeinen Formel VII
HN -B-$ONO_2$ (VII)
R
in der B und R die oben angegebenen Bedeutungen besitzen, umsetzt, oder
c) eine Verbindung der allgemeinen Formel VIII
Ar'-OH (VIII)
in der Ar' die oben angegebenen Bedeutungen hat
c1) mit einer Verbindung der allgemeinen Formel IX

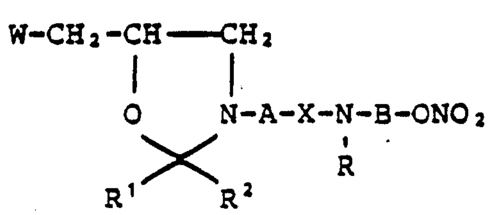 (IX)

worin A, B, X und R die oben angegebenen Bedeutungen besitzen, W Mesyloxy, Tosyloxy oder Halogen, $R_1$ Wasserstoff oder Alkyl und $R^2$ unabhaengig Wasserstoff, Alkyl oder Phenyl bedeuten oder $R^1$ und $R^2$ zusammen mit dem benachbarten Kohlenstoffatom ein Carbonyl-Radikal bilden, umsetzt, und gegebenenfalls die Gruppe Ar' in die Gruppe Ar ueberfuehrt und den Oxazolidin-Ring spaltet, oder
c2) mit einer Verbindung der allgemeinen Formel X

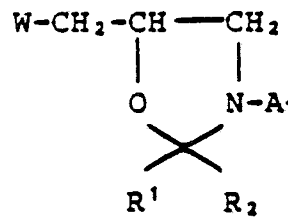
(X)

worin A, $R^1$, $R^2$, X, U und W die oben genannten Bedeutungen haben, umsetzt, den Oxazolidin-Ring spaltet und die erhaltene Verbindung der allgemeinen Formel VI, gegebenenfalls nach Uberfuehrung der Gruppe Ar' in die Gruppe Ar, mit einer Verbindung der allgemeinen Formel VII umsetzt, oder
c3) mit einer Verbindung der allgemeinen Formel XI

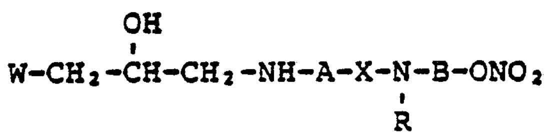
(XI)

in der A, B, W, X und R die oben angegebenen Bedeutungen besitzen, umsetzt und gegebenenfalls die Gruppe Ar' in die Gruppe Ar ueberfuehrt, oder
c4) mit einer Verbindung der allgemeinen Formel XII

OH
W-$CH_2$-CH-$CH_2$-NH-A-X-U (XII)

in der A, W, X und U die oben angegebenen Bedeutungen besitzen, zu einer Verbindung der allgemeinen Formel VI umsetzt und diese, gegebenenfalls nach Ueberfuehrung der Gruppe Ar' in die Gruppe Ar, mit einer Verbindung der allgemeinen Formel VII umsetzt,
und anschließend gewünschtenfalls die erhaltenen Verbindungen in ihre pharmakologisch unbedenklichen Salze überführt.

5. Verfahren zur Herstellung von Verbindungen der allgemeinen Formel I gemäß Anspruch 4, in der Ar eine unsubstituierte oder durch Halogen-, Cyano-, Hydroxy-, Amino-, Formyl-, Nitro-, Carboxyl-, Carbamoyl-, Trifluormethyl-, Alkyl-, Alkenyl-, Alkinyl-, Cycloalkyl-, Bicycloalkyl-, Alkanoyl-, Alkoxy-, Hydroxyalkyl-, Alkoxyalkyl-, Alkoxyalkoxyalkyl-, Alkoxyalkoxy-, Alkenyloxy-, Alkenyloxyalkyl-, Alkinyloxy-, Alkinyloxyalkyl-, Cycloalkoxy-, Alkylthio-, Alkylthioalkyl-, Morpholino-, Acylamino-, Acylaminoalkyl-, Acyloxy-, Alkoxycarbonyl-, Cycloalkyloxycarbonyl-, Alkylaminocarbonylamino-, Dialkylaminocarbonylamino-, Dialkylaminocarbonylaminoalkyl-, Dialkylaminocarbonylalkyl-, Dialkylaminocarbonylalkoxy-, wobei bei den letzteren 4 Gruppen die beiden endstaendigen Alkylgruppen zusammen mit dem Stickstoffatom auch eine cyclische Gruppe mit vier oder fuenf C-Atomen bilden koennen, Cycloalkylaminocarbonylamino-, Alkylaminocarbonylaminoalkyl-, Cycloalkylaminocarbonylaminoalkyl-, Alkoxycarbonylaminoalkyl-, Cycloalkoxycarbonylaminoalkyl-, Carbamoylalkyl-, Alkylaminocarbonylalkyl-, Cycloalkylaminocarbonylalkyl oder Alkylaminocarbonylalkoxy-Gruppen substituierte Phenylgruppe
oder eine unsubstituierte oder durch Alkyl-, Cyano-, Hydroxyalkyl-, Hydroxy-, Oxo-, Formyl-, Alkanoyl-, Alkylcarbonylamino-, Alkoxycarbonyl-oder Morpholino-gruppen substituierte Thiadiazolyl-, Naphthyl-, Indenyl-, Indolyl-, Indazolyl-, Imidazolyl-, Benzimidazolyl-, Benztriazolyl-, Benzofuranyl-, Benzodioxolyl-, Benzothiophenyl-, Benzthiazolyl-, Benzisothiazolyl-, Chinolyl-, Isochinolyl-, Benzodiazinyl-, Benzopyranyl-, Benzothiinyl-, Benzothiazinyl-, Benzothiadiazinyl-, Benzoxathiinyl-, Benzoxazolyl-, Benzisoxazolyl-, Benzoxazinyl-, Benzodioxinyl-oder Carbazolyl-Gruppe, wobei eine oder mehrere Doppelbindungen hydriert sein können, bedeutet,
A eine geradkettige oder verzweigte Alkylenkette von 1-8 C-Atomen, wobei eine -$CH_2$-Gruppe durch eine Cycloalkylengruppe von 3-7 C-Atomen ersetzt sein kann, bedeutet
X eine -C(=O)-, -S(=O)-oder -S$(=O)_2$-Gruppe bedeutet
B eine geradkettige, mono-oder bicyclische, gegebenenfalls verzweigte, gesaettigte oder ungesaettigte Alkylenkette von 1-12 C-Atomen, worin eine -$CH_2$-Gruppe durch eine Cycloalkylen-Gruppe von 3-7 C-Atomen ersetzt sein kann und/oder bis zu 2 -$CH_2$-Gruppen jeweils durch ein Sauerstoffatom oder Schwefelatom oder eine -S( = O)-oder -S( = O)$_2$-Gruppe ersetzt sein koennen, bedeutet
R Wasserstoff oder eine geradkettige oder verzweigte, gesaettigte oder ungesaettigte Alkyl-oder Nitroxyalkyl-Gruppe von 1-6 C-Atomen darstellt, oder R auch gemeinsam mit dem benachbarten Stickstoffatom und einer -CH$_2$-Gruppe der Kette B einen heteroaliphatischen Ring mit 4-6 C-Atomen aufspannen kann, bedeutet
sowie deren physiologisch vertraegliche Salze.

6. Verfahren zur Herstellung von Verbindungen der allgemeinen Formel I gemäß Anspruch 5, in der
A eine geradkettige Alkylenkette mit 1 - 3 C-Atomen oder eine verzweigte Alkylenkette mit 2 - 5 C-Atomen bedeutet,
X eine -C( = O)-, -S( = O)-oder -S( = O)$_2$-Gruppe bedeutet,
B eine geradkettige oder verzweigte Alkylenkette mit 2 - 6 C-Atomen bedeutet,
R Wasserstoff oder einen geradkettigen oder verzweigten Alkylrest mit 1 - 6 C-Atomen oder R gemeinsam mit dem benachbarten Stickstoffatom und einer CH$_2$-Gruppe der Kette B einen heteroaliphatischen Ring mit 4 - 6 C-Atomen aufspannen kann, bedeutet,
sowie deren physiologisch verträgliche Salze.

7. Arzneimittel enthaltend mindestens eine Verbindung gemäß Anspruch 1, 2 oder 3 sowie gegebenenfalls übliche Träger-und Hilfsstoffe.

8. Verwendung von Verbindungen gemäß Anspruch 1, 2 oder 3 zur Herstellung von Arzneimitteln zur Behandlung und/oder Prophylaxe von Herz-und Kreislauferkrankungen.

9. Verbindungen der allgemeinen Formel II

$$Ar{-}O{-}CH_2{-}\overset{OH}{\overset{|}{CH}}{-}CH_2{-}NH{-}A{-}X{-}\underset{\underset{R}{|}}{N}{-}B{-}OH \qquad (II)$$

worin
Ar eine substituierte oder unsubstituierte aromatische oder heteroaromatische Gruppe bedeutet,
A eine geradkettige oder verzweigte Alkylenkette von 1-8 C-Atomen, wobei eine -CH$_2$-Gruppe durch eine Cycloalkylengruppe von 3-7 C-Atomen ersetzt sein kann, bedeutet
X eine -C( = O)-, (S = O)-oder -S( = O)$_2$-Gruppe bedeutet
B eine geradkettige, mono-oder bicyclische, gegebenenfalls verzweigte, gesaettigte oder ungesaettigte Alkylenkette von 1-12 C-Atomen, worin eine -CH$_2$-Gruppe durch eine Cycloalkylen-Gruppe von 3-7 C-Atomen ersetzt sein kann und/oder bis zu 2 -CH$_2$-Gruppen jeweils durch ein Sauerstoffatom oder Schwefelatom oder eine -S( = O)-oder -S( = O)$_2$-Gruppe ersetzt sein koennen, bedeutet
R Wasserstoff oder eine geradkettige oder verzweigte, gesaettigte oder ungesaettigte Alkyl-oder Nitroxyalkyl-Gruppe von 1-6 C-Atomen darstellt, oder R auch gemeinsam mit dem benachbarten Stickstoffatom und einer -CH$_2$-Gruppe der Kette B einen heteroaliphatischen Ring mit 4-6 C-Atomen aufspannen kann, bedeutet.

10. Verfahren zur Herstellung von Verbindungen der allgemeinen Formel II

$$Ar{-}O{-}CH_2{-}\overset{OH}{\overset{|}{CH}}{-}CH_2{-}NH{-}A{-}X{-}\underset{\underset{R}{|}}{N}{-}B{-}OH \qquad (II)$$

worin
Ar eine substituierte oder unsubstituierte aromatische oder heteroaromatische Gruppe bedeutet,
A eine geradkettige oder verzweigte Alkylenkette von 1-8 C-Atomen, wobei eine -CH$_2$-Gruppe durch eine Cycloalkylengruppe von 3-7 C-Atomen ersetzt sein kann, bedeutet
X eine -C( = O)-, -S( = O)-oder -S( = O)$_2$-Gruppe bedeutet
B eine geradkettige, mono-oder bicyclische, gegebenenfalls verzweigte, gesaettigte oder ungesaettigte Alkylenkette von 1-12 C-Atomen, worin eine -CH$_2$-Gruppe durch eine Cycloalkylen-Gruppe von 3-7 C-Atomen ersetzt sein kann und/oder bis zu 2 -CH$_2$-Gruppen jeweils durch ein Sauerstoffatom oder Schwefelatom oder eine -S( = O)-oder -S( = O)$_2$-Gruppe ersetzt sein koennen, bedeutet
R Wasserstoff oder eine geradkettige oder verzweigte, gesaettigte oder ungesaettigte Alkyl-oder Nitroxyalkyl-Gruppe von 1-6 C-Atomen darstellt, oder R auch gemeinsam mit dem benachbarten Stickstoffa-

tom und einer $-CH_2$-Gruppe der Kette B einen heteroaliphatischen Ring mit 4-6 C-Atomen aufspannen kann, bedeutet,

dadurch gekennzeichnet, daß man in an sich bekannter Weise

a) eine Verbindung der allgemeinen Formel Ar'-O-Z (III)

a1) mit einem Amin der allgemeinen Formel XIII

$H_2N$-A-X- N -B-OH (XIII)
R

in der A, B, X und R die oben angegebenen Bedeutungen haben, umsetzt, oder

a2) mit einem Amin der allgemeinen Formel V zu einer Verbindung der allgmeinen Formel VI umsetzt und diese, gegebenenfalls nach Ueberfuehrung der Gruppe Ar' in die Gruppe Ar, mit einer Verbindung der allgemeinen Formel XIV

HN -B-OH (XIV)
R

in der B und R die oben angegebenen Bedeutungen besitzen, umsetzt, oder

b) eine Verbindung der allgemeinen Formel Ar'-OH-(VIII)

b1) mit einer Verbindung der allgemeinen Formel XV

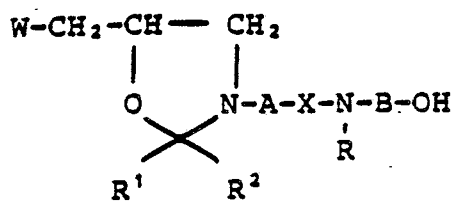
(XV)

in der A, B, $R^1$, $R^2$, W, X und R die oben angegebenen Bedeutungen haben, umsetzt und den Oxazolidin-Ring spaltet, oder

b2) mit einer Verbindung der allgemeinen Formel X zu einer Verbindung der allgemeinen Formel XVI

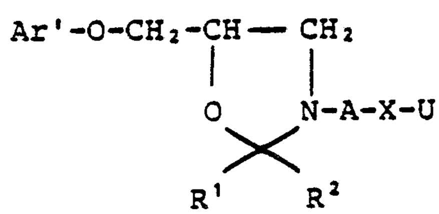
(XVI)

in der Ar', $R^1$, $R^2$, A, X und U die oben angegebenen Bedeutungen haben, umsetzt und diese, gegebenenfalls vor oder nach Ueberfuehrung der Gruppe Ar' in die Gruppe Ar, mit einer Verbindung der allgemeinen Formel XIV zu einer Verbindung der allgemeinen Formel XVII

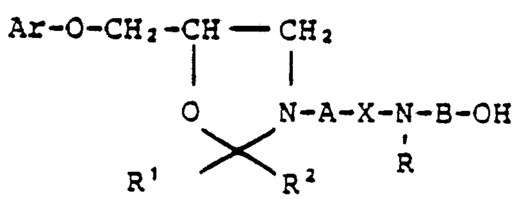
(XVII)

in der Ar, $R^1$, $R^2$, A, X, R und B die oben angegebenen Bedeutungen haben, umsetzt und dann den Oxazolidin-Ring spaltet, oder

b3) mit einer Verbindung der allgemeinen Formel X umsetzt, den Oxazolidin-Ring spaltet und die so erhaltene Verbindung der allgemeinen Formel VI, gegebenenfalls nach Ueberfuehrung der Gruppe Ar' in die Gruppe Ar, mit einer Verbindung der allgemeinen Formel XIV umsetzt, oder

b4) mit einer Verbindung der allgemeinen Formel XVIII

OH
W-$CH_2$-CH-$CH_2$-NH-A-X-N-B-OH (XVIII)
R

in der A, B, W, X und R die oben angegebenen Bedeutungen besitzen, umsetzt und gegebenenfalls die Gruppe Ar' in die Gruppe Ar ueberfuehrt, oder

b5) mit einer Verbindung der allgemeinen Formel XII zu einer Verbindung der allgemeinen Formel VI umsetzt, und diese, gegebenenfalls nach Ueberfuehrung der Gruppe Ar' in die Gruppe Ar, mit einer Verbindung der allgemeinen Formel XIV umsetzt,

c) eine Verbindung der allgemeinen Formel III mit Ammoniak oder einem geschuetzten Amin umsetzt, gegebenenfalls die Schutzgruppen abspaltet und die Gruppe Ar' in die Gruppe Ar ueberfuehrt und die entstandene Verbindung XIX

OH
Ar-O-$CH_2$- CH -$CH_2$-$NH_2$ (XIX)

in der Ar die angegebene Bedeutung hat,

c1) mit einer Verbindung der allgemeinen Formel XX

H-A'-X- N -B-OH (XX)
R

in der X, R und B die oben angegebenen Bedeutungen haben und A' die gleiche Bedeutung wie A hat, wobei aber eine -$CH_2$-Gruppe in A' durch eine C=O-Gruppe ersetzt ist, umsetzt und das entstandene Imin in situ oder anschließend reduziert, oder

c2) mit einer Verbindung der allgemeinen Formel XXI

H-A'-X-U (XXI)

in der A', X und U die oben angegebenen Bedeutungen haben, umsetzt, das entstandene Imin in situ oder anschließend weiter zur Verbindung der allgemeinen Formel VI reduziert und diese mit einer Verbindung der allgemeinen Formel XIV umsetzt.

11. Verwendung von Verbindungen gemäß Anspruch 9 als Zwischenprodukte für die Herstellung von Verbindungen gemäß Anspruch 1, 2 oder 3.

Europäisches Patentamt

# EUROPÄISCHER RECHERCHENBERICHT

Nummer der Anmeldung

EP 88 10 2169

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 200 915 (BOEHRINGER MANNHEIM) * Ansprüche * --- | 1 | C 07 C 103/50<br>C 07 D 209/08<br>C 07 D 209/34<br>C 07 D 211/46<br>A 61 K 31/16<br>A 61 K 31/40 |
| A | EP-A-0 004 532 (MERCK PATENT) * Ansprüche * --- | 1 | |
| A | FR-A-2 333 498 (IMPERIAL CHEMICAL INDUSTRIES) * Ansprüche * ----- | 1 | |
| | | | **RECHERCHIERTE SACHGEBIETE (Int. Cl.4)** |
| | | | C 07 C 103/00<br>C 07 D 209/00<br>C 07 D 211/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 31-05-1988 | PAUWELS G.R.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)